(19) Europäisches Patentamt
European Patent Office
Office européen des brevets

(11) EP 2 062 584 A1

(12) **EUROPEAN PATENT APPLICATION**
published in accordance with Art. 153(4) EPC

(43) Date of publication:
**27.05.2009 Bulletin 2009/22**

(21) Application number: **07807007.5**

(22) Date of filing: **10.09.2007**

(51) Int Cl.:
***A61K 31/663*** (2006.01) ***A61K 9/70*** (2006.01)
***A61K 47/06*** (2006.01) ***A61K 47/08*** (2006.01)
***A61K 47/10*** (2006.01) ***A61K 47/14*** (2006.01)
***A61K 47/32*** (2006.01) ***A61P 1/02*** (2006.01)
***A61P 3/14*** (2006.01) ***A61P 19/00*** (2006.01)
***A61P 19/02*** (2006.01) ***A61P 19/10*** (2006.01)
***A61P 29/00*** (2006.01) ***A61P 35/00*** (2006.01)
***A61P 35/02*** (2006.01) ***A61P 35/04*** (2006.01)

(86) International application number:
**PCT/JP2007/067597**

(87) International publication number:
**WO 2008/032678 (20.03.2008 Gazette 2008/12)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **11.09.2006 JP 2006245965**

(71) Applicant: **Kyukyu Pharmaceutical Co., Ltd.**
**Tokyo 103-0023 (JP)**

(72) Inventors:
- **YAMAZAKI, Yuuhiro**
**Imizu-shi**
**Toyama 939-0351 (JP)**
- **HAYASHI, Noriyuki**
**Imizu-shi**
**Toyama 939-0351 (JP)**
- **SAKAI, Yoshiki**
**Mishima-gun**
**Osaka 618-8585 (JP)**

(74) Representative: **Keller, Günter et al**
**Lederer & Keller**
**Patentanwälte**
**Prinzregentenstrasse 16**
**80538 München (DE)**

# (54) ADHESIVE PREPARATION

(57) The present invention provides an adhesive preparation having a plaster layer disposed on a support, the adhesive preparation comprising at least one active ingredient selected from the group consisting of a bisphosphonic acid derivative, its salt thereof and a hydrate of either of the bisphosphonic acid derivative or the salt, a solubilizer for the active ingredient, propylene glycol, a hydrogenated terpene resin, an adhesive base, and a softening agent in the plaster layer.



Printed by Jouve, 75001 PARIS (FR)

## Description

### TECHNICAL FIELD

The present invention relates to an adhesive preparation comprising a bisphosphonic acid derivative, a salt thereof or a hydrate of either of the bisphosphonic acid derivative or the salt (hereinafter sometimes collectively referred to as "bisphosphonic acid derivative or the like"). The present invention relates particularly to an adhesive preparation comprising 1-hydroxy-2-(imidazo[1,2-a]pyridin-3-yl)ethylidenebisphosph onic acid (hereinafter referred to as minodronic acid), 4-amino-1-hydroxybutane-1,1-bisphoshonic acid (referred to hereinafter as alendronic acid), 3-(N-methyl-N-n-pentylamino)-1-hydroxypropane-1,1-bisphosph onic acid (referred to hereinafter as ibandronic acid), 1-hydroxy-2-(imidazol-1-yl)-ethane-1,1-bisphosphonic acid (referred to hereinafter as zoledronic acid), 1-hydroxy-2-pyridin-3-ylethylidenediphosphonic acid (referred to hereinafter as risedronic acid), or a salt of any of the foregoing compounds or a hydrate of any of the foregoing compounds.

### BACKGROUND ART

Certain synthetic bisphosphonic acid derivatives, which are structural analogues of pyrophosphoric acid having a methanebisphosphonic acid structure and are stable also in vivo, have biological actions such as an excellent suppressive action on bone resorption and on ectopic calcification, etc. Such derivatives are useful as preparations for the treatment of diseases associated with increased bone resorption, ectopic calcification, etc., for example, osteoporosis, Paget's disease of bone, hypercalcemia accompanying malignant tumors, and bone metastasis accompanying cancer. Some of the derivatives have already been used clinically.

Known bisphosphonic acid derivatives commercially available so far or currently under development include alendronic acid, ibandronic acid, incadronic acid, etidronic acid, olpadronic acid, clodronic acid, zoledronic acid, tiludronic acid, neridronic acid, pamidronic acid, risedronic acid, minodronic acid, 1-hydroxy-3-(1-pyrrolidinyl)propylidenebisphosphonic acid, salts of any of the foregoing compounds and hydrates of any of the foregoing compounds. In particular, nitrogen atom-containing bisphosphonic acid derivatives classified as third generation have a potent suppressive action on bone resorption, and exhibit drug effect persistently for a long period once adsorbed into the bone. For example, JP-B 06-99457 describes that minodronic acid, which has a potent suppressive action on bone resorption and an excellent anti-inflammatory and analgesic antipyretic action, is useful for the treatment of increased bone resorption accompanying the following diseases: Paget's disease, hypercalcemia, bone metastasis from cancer, osteoporosis or chronic rheumatoid arthritis.

Oral preparations comprising a bisphosphonic acid derivative, a salt thereof or a hydrate of either of the bisphosphonic acid derivative or the salt, are clinically used at present, but their use is subject to the following indications: "contraindication" in "patients who cannot sit up or stand for 30 minutes or more after taking the medication", "careful administration" to "patients with upper-gastrointestinal disorders such as dysphagia, esophagitis, gastritis, duodenitis, or ulcer", and "precaution", which is attributable to low absorbability and low bioavailability upon oral administration, of "daily oral administration together with about 180 mL of water on awakening, and avoidance of eating and drinking (except water) as well as taking other oral medications for at least 30 minutes after administration". The last indication is attributed to the characteristics of the bisphosphonic acid derivative. That is, the absorption of the derivative is suppressed particularly in the presence of calcium or iron. For this reason, the influence of diet or the like must be avoided. The bisphosphonic acid derivative also has a potent gastrointestinal mucosa-damaging action. Retention or regurgitation of the drug in mucosa of the esophagus etc. must be avoided to prevent the subsequent mucosal irritation. Accordingly, the administration of the bisphosphonic acid derivative or the like is contraindicated in patients with esophageal food obstruction or in patients who cannot maintain the standing position/sitting up position after taking the medication. Because of such restriction on oral administration, patients' compliance with medication is extremely low.

Elderly patients with osteoporosis, patients with steroid-induced osteoporosis, patients with fracture, and patients with cancer often have difficulty in swallowing. It is often hard for such patients to be medicated by an oral preparation requiring multiple doses at regular time intervals under restricted condition for its administration. Further, oral administration increases the amount of the drug exposed to the gastrointestinal tract and the liver, maximum drug concentration (Cmax) in blood and renal drug excretion, thereby increasing the incidence rate of adverse drug reactions such as renal damage in addition to gastrointestinal disorders particularly in the esophagus and gastric mucosa.

Accordingly, from the viewpoint of improving the quality of life of patients having difficulty in oral administration, oral preparations to be intermittently administered once a week or once a month, in place of oral preparations to be administered once a day, have been developed and examined to reduce and circumvent the problems described above. However, there are still many problems, one of which is an increased single dose. Injections to be administered once a month to once every 12 months have also been developed and examined. However, the administration of an injection causes pain, leads to a rapid increase in blood drug concentration possibly resulting in the incidence of adverse drug

reactions such as renal damage, and takes 15 minutes to 2 hours straight via intravenous route. Patients' compliance with medication therefore remains low. Further, there is concern that oral preparations to be intermittently administered in a large single dose or injections increase the incidence rate of hypocalcemia and jawbone necrosis due to a rapid increase in blood drug concentration after administration.

Meanwhile, from the concept of drug delivery system intended for optimization of therapy, transdermally absorbable preparations with sustained drug effects and reduced adverse drug effects attract attention and are under intense study. These effects make a clear distinction from those of the conventional external preparations. Specifically, parenteral administration routes, particularly a transdermal drug delivery system typically utilizing ointments and adhesive preparations, attract attention as effective routes of drug administration. In transdermal administration, a drug gradually enters into subcutaneous capillaries directly without passing through the gastrointestinal tract. Thus, the drug can reach the target site with avoiding any hepatic first-pass effect, i.e. , hardly undergoing metabolism and decomposition, and without generating gastrointestinal disorders. Thereby, the transdermal administration increases the bioavailability of the drug. Based on this, it can be expected that stable blood pharmacokinetics can be achieved even in patients with gastrointestinal disorders or dysphasia, and that the onset of renal damage, hypocalcemia, and jawbone necrosis can be also circumvented.

The "transdermally absorbable preparations (transdermal systems)" are the preparations which are designed to deliver their active ingredient through the skin to circulating blood in the whole body after applied to the skin, and are referred to in the present invention as "adhesive preparations".

However, the adhesive preparations are generally poor in transdermal drug absorption due to skin barrier function. Thus, it is often hard for the adhesive preparations in a practical and limited area size for application to transdermally deliver a necessary amount of the drug to exhibit drug effect. The largest barrier in transdermal drug absorption is the stratum corneum, which is a complex structure of small corneocyte residuals separated from each other in between the extracellular lipid matrix. In the stratum corneum, drug permeation is far lower than in mucosa of the mouth or stomach. The adhesive preparations also have many problems in drug stability, skin irritancy, persistency, effect, safety (incidence of adverse drug reactions), adhesiveness, and uncomfortable feeling.

For example, US Patent No.5133972 discloses an invention directed to a topically administrable pharmaceutical preparation, comprising a particular methanediphosphonic acid represented by the following formula (I) or a pharmaceutically acceptable salt thereof:

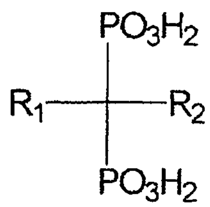

(I)

(wherein ... Het1 is unsubstituted or substituted, monocyclic, 5- or 6-memberred monoaza-, diaza- or thiaza-aryl that is bonded via a ring carbon atom, or wherein $R_1$ is ... or hydroxy, $R_2$ is $-A-R_3$, in which A is alkylene and $R_3$ is Het2, which is Het1 bonded via a ring carbon or ring nitrogen atom, or, ... (as to further detailed definitions, see the above-mentioned U.S. Patent specification)).

JP-A 53-34930 discloses an invention directed to a composition particularly suitable for localized treatment, including transdermal administration, of abnormal mobilization and deposition of calcium phosphates in human and lower-animal tissues, the composition comprising a safe and effective amount of an organic phosphonate compound and a carrier containing a safe and effective amount of an organic sulfoxide compound as a specific permeability enhancer. Decylmethylsulfoxide is described to be most preferable as the organic sulfoxide compound in the composition. However, the preparation compounded with decylmethylsulfoxide described in the Examples was insufficient in transdermal permeability for practical use as an adhesive preparation.

WO 03/068241 discloses a bisphosphonic acid derivative-containing transdermal preparation comprising a bisphosphonic acid derivative or a pharmaceutically acceptable salt, a solubilizer therefor and an amphiphilic solubilizing aid ingredient therefor. The preparation compounded with N-methyl-pyrrolidone as an amphiphilic solubilizing aid ingredient described in the Examples was good in transdermal permeability, but its permeability was still low. The permeability was not high enough even if the amount of a drug was increased in the adhesive preparation. Thus, the adhesive preparation cannot be expected much as an efficient one and is not suitable for industrial production on a large scale because of its poor coatability in a pharmaceutical production process.

## SUMMARY OF INVENTION

### TECHNICAL PROBLEM

As described above, the bisphosphonic acid derivative or the like possibly induces the onset of skin irritation (erythema, edema, pruritus, rash, pigmentation, etc.) due to its characteristics. Further, its extremely low transdermal absorbability makes it difficult to sustain such a sufficient blood concentration of the bisphosphonic acid derivative or the like as to exhibit its effect via absorption through the skin. Furthermore, the bisphosphonic acid derivative or the like is poor in coatability (spreadability) for the production of adhesive preparations because of its physical properties, and thus has a problem in that it is not suitable for industrial production on a large scale. No adhesive preparations that comprise the bisphosphonic acid derivative or the like and that can solve all such problems have been put into practical use.

Under these circumstances, an object of the present invention is to provide adhesive preparations such as tapes and plasters that comprise a bisphosphonic acid derivative, a salt thereof or a hydrate of either of the bisphosphonic acid derivative or the salt, and that can minimize burden on patients without compromising patients' compliance with medication even when administered over a long period of time.

### SOLUTION TO PROBLEM

To solve the problems, the present inventors extensively examined a suitable combination and compounding ratio of a base for external use that can increase the transdermal permeation of the bisphosphonic acid derivative or the like, can sustain such a sufficient blood concentration of the bisphosphonic acid derivative or the like over a prolonged period as to exhibit its effect with reduced skin irritation, and can give good coatability for the production of adhesive preparations.

As a result, the present inventors found that an adhesive preparation having a plaster layer formed by a combination of a bisphosphonic acid derivative or the like as an active ingredient and a base for external use comprising propylene glycol, a hydrogenated terpene resin, an adhesive base, and a softening agent is excellent in transdermal absorption, shows high permeability per unit area, can sustain such a sufficient blood drug concentration over a prolonged period as to exhibit drug effect, is excellent in coatability for the production of adhesive preparations, is excellent in adhesiveness, and has less skin irritancy. Thus, the present invention was completed.

In any of the patent documents described above, there is no description on the combination of a bisphosphonic acid derivative, a salt thereof or a hydrate of either of the bisphosphonic acid derivative or the salt and a special base for external use according to the present invention, or on the preferable compounding ratio thereof. There is no description and suggestion of properties of an adhesive preparation prepared by the special combination, either. The present invention can never be predicted from the conventional art.

That is, the present invention which can solve the problems described above relates to an adhesive preparation having the following constitution:

(1) An adhesive preparation having a plaster layer disposed on a support, which is characterized by that the adhesive preparation comprises at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, anadhesivebase, and a softening agent in the plaster layer.

(2) The adhesive preparation according to the above-mentioned (1), wherein the softening agent is at least one member selected from the group consisting of polybutene, liquid paraffin, and light liquid paraffin.

(3) The adhesive preparation according to the above-mentioned (1), wherein the content of the active ingredient in one adhesive preparation or in the adhesive preparations applied once is 0.01 mg to 30 mg.

(4) The adhesive preparation according to the above-mentioned (3), wherein the sum of an area under blood concentration-time curve (AUC) value of the bisphosphonic acid derivative for 1 month is 5 ng·hr/mL to 5 μg·hr/mL.

(5) The adhesive preparation according to the above-mentioned (1), wherein the relative standard deviation of the mass of the plaster layer in one adhesive preparation is 5% or less when the number of samples is 10.

(6) The adhesive preparation according to the above-mentioned (1), wherein the thickness of the plaster layer is 20 μm to 300 μm.

(7) The adhesive preparation according to the above-mentioned (1), wherein the active ingredient is at least one member selected from the group consisting of minodronic acid, alendronic acid, ibandronic acid, zoledronic acid, risedronic acid, incadronic acid, etidronic acid, olpadronic acid, clodronic acid, tiludronic acid, neridronic acid, pamidronic acid, salts of any of the foregoing compounds and hydrates of any of the foregoing compounds.

(8) The adhesive preparation according to the above-mentioned (1), wherein the active ingredient is at least one member selected from the group consisting of alendronic acid, ibandronic acid, zoledronic acid, risedronic acid,

salts of any of the foregoing compounds and hydrates of any of the foregoing compounds.

(9) The adhesive preparation according to the above-mentioned (1), wherein the active ingredient is minodronic acid, a salt thereof or a hydrate of either of minodronic acid or the salt.

(10) The adhesive preparation according to the above-mentioned (1), which further comprises at least one member selected from the group consisting of polyoxyethylene alkyl ether, polyoxyethylene alkyl ether phosphate or a salt thereof, oleyl alcohol, and sorbitan fatty acid ester.

(11) The adhesive preparation according to the above-mentioned (10),wherein the polyoxyethylene alkyl ether is polyoxyethylene behenyl ether, the polyoxyethylene alkyl ether phosphate is tripolyoxyethylene cetyl ether phosphate, and the sorbitan fatty acid ester is sorbitan sesquioleate.

(12) The adhesive preparation according to the above-mentioned (2), which further comprises isopropyl myristate.

(13) The adhesive preparation according to the above-mentioned (1), which further comprises at least one member selected from the group consisting of a rosin resin, a petroleum resin, and another terpene resin.

(14) The adhesive preparation according to the above-mentioned (13), wherein the rosin resin is glycerol ester of hydrogenated rosin and/or super light-colored rosin ester.

(15) The adhesive preparation according to the above-mentioned (1), wherein the adhesive base is at least one member selected from the group consisting of a rubber adhesive base, an acrylic adhesive base, and a silicone adhesive base.

(16) The adhesive preparation according to the above-mentioned (15), wherein the rubber adhesive base is at least one member selected from the group consisting of a styrene-isoprene-styrene block copolymer, a styrene-butadiene-styrene block copolymer, a styrene-ethylene-styrene block copolymer, a styrene-butylene-styrene block copolymer, polyisoprene, polyisobutylene, and an ethylene-vinyl acetate copolymer.

(17) The adhesive preparation according to the above-mentioned (15), wherein the rubber adhesive base is a styrene-isoprene-styrene block copolymer.

(18) The adhesive preparation according to the above-mentioned (1), wherein 0.1 to 10 parts by mass of the active ingredient, 1 to 50 parts by mass of propylene glycol, 10 to 80 parts by mass of the adhesive base, 5 to 60 parts by mass of the hydrogenated terpene resin, and 5 to 60 parts by mass of the softening agent are contained in 100 parts by mass of the plaster layer.

(19) The adhesive preparation according to the above-mentioned (18), wherein the adhesive base is a rubber adhesive base and is contained in an amount of 10 to 50 parts by mass, and the hydrogenated terpene resin is contained in an amount of 10 to 60 parts by mass.

(20) The adhesive preparation according to the above-mentioned (18), wherein the adhesive base is an acrylic adhesive base and is contained in an amount of 20 to 80 parts by mass, and the hydrogenated terpene resin is contained in an amount of 5 to 30 parts by mass.

(21) The adhesive preparation according to the above-mentioned (18), wherein the adhesive base is a silicone adhesive base and is contained in an amount of 20 to 80 parts by mass, and the hydrogenated terpene resin is contained in an amount of 5 to 30 parts by mass.

(22) The adhesive preparation according to the above-mentioned (1), wherein the solubilizer for the active ingredient is water or an aqueous alkaline solution.

(23) The adhesive preparation according to the above-mentioned (1), which is applied once a day, once every two days, once every 3 to 4 days, once a week to once every three weeks, or once a month to once every three months and is stuck for 6 hours to 7 days after being once applied.

(24) The adhesive preparation according to the above-mentioned (1), which is applied once a day, once every two days, once a week, or once a month and is stuck for 24 hours to 4 days after being once applied.

(25) The adhesive preparation according to the above-mentioned (1), which is a preventive and/or therapeutic preparation for at least one disease selected from osteoporosis, bone metastasis from cancer, hypercalcemia, multiple myeloma, Paget's disease, periodontal diseases, osteoarthritis, and rheumatoid arthritis.

(26) An adhesive preparation containing 0.5 to 5 parts by mass of minodronic acid, a salt thereof or a hydrate of either of minodronic acid or the salt as an active ingredient, 2 to 15 parts by mass of a combination of propylene glycol and polyoxyethylene behenyl ether, 20 to 60 parts by mass of a combination of a hydrogenated terpene resin and glycerol ester of hydrogenated rosin, 10 to 40 parts by mass of polybutene, liquid paraffin or light liquid paraffin, and 15 to 35 parts by mass of a styrene-isoprene-styrene block copolymer in 100 parts by mass of a plaster layer,

the adhesive preparation satisfying any of the following conditions (1) to (3):

(1) the content of the active ingredient in one adhesive preparation or in the adhesive preparations applied once is 0.1 mg to 10 mg,

(2) the sum of an area under blood concentration-time curve (AUC) value of minodronic acid for 1 month is 30 ng·hr/mL to 5 μg·hr/mL, and

(3) the relative standard deviation of the mass of the plaster layer in one adhesive preparation is 5% or less when the number of samples is 10.

(27) An adhesive preparation containing 0.5 to 5 parts by mass of minodronic acid, a salt thereof or a hydrate of either of minodronic acid or the salt as an active ingredient, 2 to 15 parts by mass of a combination of propylene glycol and polyoxyethylene behenyl ether, 20 to 60 parts by mass of a combination of a hydrogenated terpene resin and glycerol ester of hydrogenated rosin, 10 to 40 parts by mass of polybutene, liquid paraffin or light liquid paraffin, and 15 to 35 parts by mass of a styrene-isoprene-styrene block copolymer in 100 parts by mass of a plaster layer,

the adhesive preparation satisfying any of the following conditions (1) to (3):

(1) the content of the active ingredient in one adhesive preparation or in the adhesive preparations applied once is 0.1 mg to 10 mg,

(2) the sum of an area under blood concentration-time curve (AUC) value of minodronic acid for 1 month is 30 ng·hr/mL to 500 ng·hr/mL, and

(3) the relative standard deviation of the mass of the plaster layer in one adhesive preparation is 5% or less when the number of samples is 10.

The present invention also relates to:

(28) use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent as an adhesive preparation;

(29) use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent as an adhesive preparation for prevention and/or treatment of bone diseases;

(30) use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent for the production of an adhesive preparation; and

(31) use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent for the production of an adhesive preparation for prevention and/or treatment of bone diseases.

Further, the present invention relates to:

(32) a method of applying, to the skin, a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent, and

(33) a method of preventing and/or treating bone diseases, which comprises applying, to the skin, a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent.

ADVANTAGEOUS EFFECTS OF INVENTION

The present invention can provide the adhesive preparation which can sustain the transdermal absorption of a bisphosphonic acid derivative, a salt thereof or a hydrate of either of the bisphosphonic acid derivative or the salt into the body in an efficient and stable manner and can be industrially produced in a stable way due to its plaster layer having excellent coatability.

BRIEF DESCRIPTION OF DRAWINGS

FIG. 1 is a schematic graph showing one example of blood concentration-time curve.
FIG. 2 is a schematic graph showing another example of blood concentration-time curve.
FIG. 3 is a schematic graph showing another example of blood concentration-time curve.

DESCRIPTION OF EMBODIMENTS

The adhesive preparation of the present invention encompasses preparations usually referred to as tapes , plasters , cataplasms and patches. Herein, the "adhesive preparation" shall be broadly interpreted. The adhesive preparation includes, for example, an adhesive preparation comprising a plaster layer containing a drug, a support for backing the plaster layer, and a release film for protecting the surface of the plaster layer at the opposite side of the support. The plaster layer may be either a laminate plaster layer having a plurality of drug-containing layers and drug-free layers, or a plaster layer having a drug-containing layer and a drug-free layer on the periphery of the drug-containing layer (frame type).

The "support" used as a backing (backing member) of the plaster layer is in the form of a stretchable or non-stretchable sheet. Examples include a film made of polyester, polypropylene, polyethylene, an ethylene-vinyl acetate copolymer, vinylon, polyvinyl chloride, nylon or polyurethane; a nonwoven fabric; a woven fabric; and a laminate comprising a combination of two or more of the foregoing members. Further, a laminate comprising a combination of two or more members selected from a film, a nonwoven fabric and a woven fabric may be manufactured by laminating these members with one another via an adhesive or through thermal fusion bonding.

The "release film" for protecting the plaster layer includes, for example, paper, polyester film, polyethylene film, polypropylene film, etc. each of which has been subjected to release treatment.

The bisphosphonic acid derivative or the like as the active ingredient in the adhesive preparation of the present invention includes, for example, minodronic acid, alendronic acid, ibandronic acid, zoledronic acid, risedronic acid, incadronic acid, etidronic acid, olpadronic acid, clodronic acid, tiludronic acid, neridronic acid, pamidronic acid, salts of any of the foregoing compounds and hydrates of any of the foregoing compounds. Among them, one or more members can be used.

In the present invention, a salt of the bisphosphonic acid derivative is preferably toxicity-free and water-soluble. Suitable examples of the salt include salts of alkali metals (potassium, sodium and the like), salts of alkaline earth metals (calcium, magnesium and the like), ammonium salts, and salts of pharmaceutically acceptable organic amines (tetramethyl ammonium, triethylamine, methylamine, dimethylamine, cyclopentylamine, benzylamine, phenethylamine, piperidine, monoethanolamine, diethanolamine, tris(hydroxymethyl) methylamine, lysine, arginine, N-methyl-D-glucamine and the like).

The bisphosphonic acid derivative or the like of the present invention may be a hydrate. Specific examples of the hydrate include minodronic acid hydrate, alendronate sodium hydrate, ibandronate sodium hydrate, zoledronic acid hydrate, risedronate sodium hydrate, incadronate disodium hydrate, etidronate disodium, olpadronic acid, clodronate sodium hydrate, tiludronate disodium, neridronic acid, and pamidronate disodium hydrate, from which one or more can be selected for use.

The active ingredient in the present invention is preferably at least one member selected from the group consisting of alendronic acid, ibandronic acid, zoledronic acid, risedronic acid, salts of any of the foregoing compounds and hydrates of any of the foregoing compounds. The active ingredient is also preferably minodronic acid, a salt thereof or a hydrate of either of minodronic acid or the salt. Inter alia, the active ingredient is more preferably minodronic acid hydrate, alendronate sodium hydrate, ibandronic acid, zoledronic acid hydrate, or risedronate sodium hydrate. The active ingredient is even more preferably ibandronic acid, zoledronic acid hydrate, or minodronic acid hydrate because of its potent suppressive action on bone resorption.

The present inventors focused attention on the area under blood concentration-time curve (AUC) value in order to sustain such a sufficient blood drug concentration over a prolonged period as to exhibit drug effect. Drugs with a high AUC value immensely can reduce burden on patients, since they do not always have to be administered daily to even patients with diseases (for example, osteoporosis) requiring a long-term medication.

The present inventors found the following relationship between the size of the AUC value and the manifestation of drug effect, from results of nonclinical and clinical tests using minodronic acid as the bisphosphonic acid derivative. That is, minodronic acid is accumulated in the bone for a long time via strong binding to hydroxyapatite of the bone. Accumulated minodronic acid is incorporated into osteoclasts and then suppresses bone resorption by inducing apoptosis of the osteoclasts, etc. Thereby, bone turnover is suppressed and bone mineral content (bone mineral density) is increased. That is, the AUC value after administration correlates with the amount of minodronic acid accumulated in the bone, which further correlates with the amount of minodronic acid incorporated into osteoclasts. The inventors found that as the amount of minodronic acid incorporated into the bone increases, bone destruction gets suppressed, and that as a result, bone turnover is suppressed, thereby resulting in increase in bone formation and bone mineral content. It was also found that by the action of increasing bone mineral content, bone strength is increased and thereby the incidence

rate of bone fracture is decreased. From these findings, it can be said that due to the suppressive action on bone resorption, drugs with a high AUC value have an enhanced effect in increasing bone mineral content and are more effective in declining the incidence rate of bone fracture.

Because an existing oral preparation or injection comprising a bisphosphonic acid derivative or the like as the active ingredient shows a low AUC value when administered in a single dose, it requires frequent administration to achieve a high AUC value, that is, to increase the bone mineral content. The oral preparation when administered in a large single dose increases the risk of the onset of gastrointestinal disorder. The injection or a large dose of the oral preparation each followed by a rapid increase in blood drug concentration, increases the risk of the onset of renal damage, hypocalcemia, and jawbone necrosis. Such an injection requires long intravenous drip infusion. Thus, it has been difficult that the oral preparation or the injection achieves a high AUC value.

Even by single application, the adhesive preparation of the present invention can sustain the transdermal absorption of the active ingredient and can show a stable blood pharmacokinetics pattern not accompanied by a rapid increase in blood drug concentration, thereby showing a high AUC value. Such an adhesive preparation can be intermittently administered at long intervals to patients, who can feel much less burden and show more excellent compliance with medication.

Ibandronic acid, zoledronic acid (or its hydrate), and minodronic acid (or its hydrate) classified as the third-generation bisphosphonic acid derivatives have the most potent suppressive action on bone resorption as a pharmacological activity, among the existing bisphosphonic acid derivatives. They are almost equal in strength of the suppressive action on bone resorption to each other. Meanwhile, alendronate sodium (or its hydrate) or risedronate sodium (or its hydrate) has a weaker suppressive action on bone resorption, which is about 1/5 to 1/10 relative to that of the above third-generation bisphosphonic acid derivatives. The AUC value of alendronate sodium or risedronate sodium should be about 5 to 10 times as high as that of ibandronic acid, zoledronic acid, and minodronic acid, etc. to equally give such a pharmacological activity.

The effective blood concentration of the bisphosphonic acid derivative for human can be extrapolated from results of various clinical tests, animal experiments, etc. For example, when minodronic acid hydrate was used, the sum of the AUC value of minodronic acid per month that gives a suppressive effect on osteoporosis was about 5 ng·hr/mL or more, from the minimum effective dose in oral administration in rat models of osteoporosis.

The sum of the AUC value in humans per month can be estimated to be from 1-fold to 100-fold relative to that in rats.

Alendronic acid (alendronate sodium hydrate) or risedronic acid (risedronate sodiumhydrate) has the 1/5 to 1/10 suppressive action on bone resorption relative to that of the above third-generation bisphosphonic acid derivatives, and thus the AUC value of alendronic acid or risedronic acid should be 5 to 10 times as high as that of the above third-generation bisphosphonic acid derivatives to equally give drug effect.

From these results, it can be rationally estimated that the sum of the AUC value for 1 month to give the intended effect without adverse drug reactions, is about 5 ng·hr/mL to about 500 ng·hr/mL for minodronic acid, zoledronic acid or ibandronic acid, or about 25 ng·hr/mL to about 5 μg·hr/mL for alendronic acid or risedronic acid.

Accordingly, the sum of the AUC value for 1 month in humans, that is, the sum of the area under blood concentration-time curve value of the bisphosphonic acid derivative for 1 month, is preferably about 5 ng·hr/mL to about 5 μg·hr/mL in the present invention. In a clinical test where minodronic acid hydrate was orally administered once daily to patients with osteoporosis , the AUC value of about 1 ng·hr/mL or more per day was effective in the treatment of osteoporosis. In consideration of adverse drug reactions, the AUC value of about 1 ng·hr/mL to about 500 ng·hr/mL per day is preferable for the treatment of osteoporosis. From this result, the sum of the AUC value of minodronic acid for 1 month in humans is more preferably about 30 ng·hr/mL to about 5 μg·hr/mL, even more preferably about 30 ng·hr/mL to about 500 ng·hr/mL, in order to treat osteoporosis.

The sum of the AUC value for 1 month refers to the following: for example, in a blood concentration-time curve having one cycle of the rise-fall of blood drug concentration for 1 month as shown schematically in FIG. 1, the sum of the AUC value for 1 month corresponds to the AUC value (S) in one cycle; for example, in a blood concentration-time curve having 4 cycles of the rise-fall of blood drug concentration for 1 month as shown schematically in FIG. 2, the sum of the AUC value for 1 month corresponds to the sum of the AUC values ($S_1$ to $S_4$) in the respective cycles. In the latter case, however, there can be the case where before the blood drug concentration falls to 0, it picks up. FIG. 3 shows an exemplary blood concentration-time curve having plural cycles of the rise-fall of blood drug concentration for 1 month, in which the blood drug concentration picks up before it falls to 0. In this case, the sum of the AUC value for 1 month corresponds to $S_5$.

In a preferable aspect, the adhesive preparation of the present invention is applied once daily (application frequency of once a day), once every 2 days, once every 3 to 4 days, once a week to every 3 weeks, or once a month to every 3 months to achieve the preferable sum of the AUC value for 1 month. In the case of intermittent application, the adhesive preparation may be applied repeatedly for 2 to 7 days and may not be applied for 21 to 26 days per month. More preferably, the adhesive preparation is applied once a day, once every 2 days, once every 3 to 4 days, once a

week, once every 2 weeks, or once a month. Even more preferably, the adhesive preparation is applied once a day, once every 2 days, once a week, or once a month. In consideration of skin irritation, bathing, etc., the adhesive preparation once applied is stuck over a period of usually about 6 hours to about 7 days, preferably about 6 hours to about 4 days, more preferably about 24 hours to about 4 days, even more preferably about 24 hours to about 3 days. The more specific period is preferably about 6 hours, about 12 hours, about 24 hours (1 day), about 48 hours (2 days), about 72 hours (3 days), or about 96 hours (4 days), more preferably about 12 hours, about 24 hours (1 day), about 48 hours (2 days), about 72 hours (3 days), or about 96 hours (4 days), even more preferably about 24 hours (1 day), about 48 hours (2 days), about 72 hours (3 days), or about 96 hours (4 days). In a preferable aspect, the adhesive preparation of the present invention is applied once a week or once a month and stuck over a period of about 24 hours (1 day) to about 3 days.

The application site of the adhesive preparation includes, but is not limited to, a region behind the ear, an arm, abdomen (preferably the lower abdomen etc.), chest, back, waist, hip, leg (preferably, inside of the thigh, calf, etc.), and affected areas (knee, oral cavity, etc.). One sheet or two to four sheets of the adhesive preparation are applied to the same site or different sites. When plural sheets are applied, their positions are not particularly limited, but the sheets are applied preferably in symmetric positions. For circumventing skin irritation, it is preferable that the sheets are not applied continuously at the same position.

In consideration of achieving the preferable sum of the AUC value for 1 month in humans, the content of the active ingredient (bisphosphonic acid derivative or the like) is preferably about 0.01 mg to about 30 mg, more preferably about 0.1 mg to about 10 mg, in one adhesive preparation or in the adhesive preparations applied once, although it varies with the application period, the base for external use, the compounding amounts thereof, etc. Particularly, when the adhesive preparation is stuck for 48 hours, the bisphosphonic acid derivative or the like in one adhesive preparation or in the adhesive preparations applied once is contained preferably in an amount of about 0.05 mg to about 5 mg, more preferably about 0.1 mg to about 3 mg, even more preferably about 0. 2 mg to about 2 mg.

The content of the active ingredient in the adhesive preparations applied once means the total amount of the active ingredient in a plurality of the adhesive preparations applied at the same period to the skin of the same human.

In the adhesive preparation of the present invention, the content of the bisphosphonic acid derivative or the like is preferably about 0.1 parts by mass to about 10 parts by mass, more preferably about 0.5 parts by mass to about 5 parts by mass, based on 100 parts by mass of the whole of the plaster layer.

The adhesive preparation of the present invention comprises propylene glycol, a hydrogenated terpene resin , an adhesive base , and a softening agent in the plaster layer.

In addition to propylene glycol, the adhesive preparation may comprise at least one member selected from the group consisting of polyoxyethylene alkyl ether, polyoxyethylene alkyl ether phosphate or a salt thereof, oleyl alcohol, and sorbitan fatty acid ester.

In addition to the hydrogenated terpene resin, the adhesive preparation may comprise at least one resin selected from the group consisting of a rosin resin, a petroleum resin, and another terpene resin.

The adhesive base is preferably at least one member selected from the group consisting of a rubber adhesive base, an acrylic adhesive base, and a silicone adhesive base.

The softening agent is preferably at least one member selected from the group consisting of polybutene, liquidparaffin, and light liquid paraffin. As the softening agent, isopropyl myristate may also be contained in the adhesive preparation.

In the present invention, the polyoxyethylene alkyl ether which maybe contained in addition to propylene glycol includes polyoxyethylene lauryl ether, polyoxyethylene stearyl ether, polyoxyethylene cetyl ether, polyoxyethylene nonyl phenyl ether, polyoxyethylene octyl phenyl ether, polyoxyethylene oleyl ether, polyoxyethylene cetostearyl ether, and polyoxyethylene behenyl ether, among which polyoxyethylene behenyl ether is particularly preferable. The polyoxyethylene alkyl ether phosphate or a salt thereof includes, for example, sodium dipolyoxyethylene lauryl ether phosphate, sodium dipolyoxyethylene oleyl ether phosphate, tripolyoxyethylene lauryl ether phosphate, tripolyoxyethylene cetyl ether phosphate, or a salt of any of the foregoing compounds, among which tripolyoxyethylene cetyl ether phosphate or a salt thereof is particularly preferable. The sorbitan fatty acid ester includes, for example, sorbitan monooleate, sorbitan trioleate, sorbitan monolaurate, sorbitan sesquioleate, sorbitan monostearate, sorbitan tristearate, sorbitan monopalmitate, sorbitan sesquistearate, sorbitan monoisostearate, and sorbitan sesquiisostearate, among which sorbitan sesquioleate is particularly preferable.

The plaster layer in the adhesive preparation of the present invention may be compounded with a suitable combination of propylene glycol and at least one member selected from the group consisting of polyoxyethylene alkyl ether, polyoxyethylene alkyl ether phosphate or a salt thereof, oleyl alcohol, and sorbitan fatty acid ester. The combination is more preferably a combination of propylene glycol and polyoxyethylene behenyl ether.

In the present invention, not only propylene glycol but also polyoxyethylene alkyl ether, polyoxyethylene alkyl ether phosphate or a salt thereof, oleyl alcohol, and sorbitan fatty acid ester exhibit a transdermal absorption-promoting action as well as an emulsifying action and a softening action.

In the present invention, a rubber adhesive base, an acrylic adhesive base, a silicone adhesive base, etc. can

be used as the adhesive base.

The rubber adhesive base includes, for example, ABA block copolymers (for example, styrene-isoprene-styrene block copolymer (SIS), styrene-butadiene-styrene block copolymer (SBS), styrene-ethylene-styrene block copolymer (SES), and styrene-butylene-styrene block copolymer (SBRS)), polyisoprene, polyisobutylene, and an ethylene-vinyl acetate copolymer. These rubber adhesive bases may be used alone or as a combination thereof and incorporated into the plaster layer. The rubber adhesive base is preferably a styrene-isoprene-styrene block copolymer.

The acrylic adhesive base includes, for example, copolymers of an alkyl (meth)acrylate and another functional monomer. The alkyl (meth)acrylate includes, for example, n-butyl acrylate, n-butyl methacrylate, hexyl acrylate, 2-ethylbutyl acrylate, isooctyl acrylate, 2-ethylhexyl acrylate, 2-ethylhexyl methacrylate, undecyl acrylate, dodecyl acrylate, tridecyl acrylate, and tridecyl methacrylate. The functional monomer includes monomers having, in their molecule, at least one unsaturated double bond involved in copolymerization reaction and having, in their side chain, a functional group such as a carboxyl group, a hydroxyl group, a sulfoxyl group, an amino group, an amide group or an alkoxyl group. The acrylic adhesive base is preferably a copolymer of an alkyl (meth)acrylate and (meth)acrylic acid.

The silicone adhesive base includes, for example, dimethyl silicone rubber, phenyl methyl silicone rubber, phenyl vinyl methyl silicone rubber, and fluorosilicone rubber, among which dimethyl silicone rubber is particularly preferable.

The plaster layer in the adhesive preparation of the present invention may be compounded with a suitable combination of a hydrogenated terpene resin and at least one member selected from a rosin resin, a petroleum resin, and another terpene resin. The preferable resin used in combination with the hydrogenated terpene resin is the rosin resin.

In the present invention, the hydrogenated terpene resin is a resin having an average molecular weight of 600 to 750, manufactured by hydrogenating almost 100% of the unsaturated bonds of a terpene resin obtained from pine tree-derived terpene oil, etc. as a main raw material. Commercial products of such a hydrogenated terpene resin include, for example, CLEARON P105, P115, P125, M105, M115, K100 and K110 (manufactured by Yasuhara Chemical Co., Ltd.). In the present invention, this hydrogenated terpene resin can act as a tackifier and also contribute to improvement of transdermal permeability.

In the present invention, the rosin resin which may be contained in addition to the hydrogenated terpene resin refers to a derivative obtained from rosin. Rosin is a resin obtained by removing essential oils from secreted substances of respective plants (Pinaceae) of the genus *Pinus.* The "derivative" means a hydrogenated product, a polymer, a super light-colored rosin obtained by making rosin light-colored, or a glycerol, pentaerythritol or methyl alcohol ester of any of the foregoing compounds. Commercial products of the rosin resin include, for example, Ester Gum, Pensel, Super Ester, Hypale, Pinecrystal (manufactured by Arakawa Chemical Industries, Ltd.), and the like.

The rosin resin is preferably glycerol ester of hydrogenated rosin and/or super light-colored rosin ester. The glycerol ester of hydrogenated rosin is obtained by hydrogenating unsaturated bonds of rosin, followed by esterifying with glycerin. Commercial products of such glycerol ester of hydrogenated rosin include, for example, Ester Gum H, Pinecrystal KE311 (manufactured by Arakawa Chemical Industries, Ltd.), and the like. This glycerol ester of hydrogenated rosin can act as a tackifier and also contribute to improvement of coatability.

The petroleum resin includes, for example, an alicyclic saturated hydrocarbon resin, an aliphatic hydrocarbon resin, an aliphatic aromatic copolymer resin, and an aliphatic saturated hydrocarbon resin, among which the aliphatic hydrocarbon resin is preferable.

Another terpene resin includes resins obtained from pine tree-derived terpene oil, etc. as a main raw material. Its examples include a terpene resin, an $\alpha$-pinene resin, a $\beta$-pinene resin, etc. individually obtained from a different raw material and further include a modified terpene resin obtained by reacting the above resins with other fats and oils, and a terpene phenol resin. Commercial products include, for example, YS Resins PX1250, PX1150, D115, PX1150N, TO125, TO115, TR105, YS Polyster U115, T115 (manufactured by Yasuhara Chemical Co., Ltd.), and the like.

The rosin resin, petroleum resin, and another terpene resin have a tackifying action and can be used as a tackifier.

In the present invention, the softening agent is a liquid or a liquid polymer which is compatible with an adhesive base and is incorporated to increase plasticity and processability by adding softness. The softening agent includes, for example, fats and oils such as olive oil and rapeseed oil; hydrocarbons such as squalane, squalene, petrolatum, liquid paraffin, and light liquid paraffin; fatty acids such as myristic acid and stearic acid; higher fatty acid esters such as isopropyl myristate and oleyl oleate; and synthetic resin softening agents such as polybutene and liquid isoprene rubber. In the present invention, at least one member selected from the group consisting of polybutene, liquid paraffin, light liquid paraffin, and isopropyl myristate is preferably contained as the softening agent in the plaster layer to achieve adhesiveness necessary for efficient absorption of the active ingredient. More preferably, at least one member selected from the group consisting of polybutene, liquid paraffin, and light liquid paraffin is contained as the softening agent in the plaster layer. It will be understood that in one aspect of the present invention, the softening agent may be composed of only one member selected from the group consisting of polybutene, liquid paraffin, and light liquid paraffin.

The above-mentioned polybutene is a colorless, transparent, tasteless and viscous liquid polymer primarily comprising isobutene. The polybutene preferably has an average molecular weight of 720 to 1350 and a kinematic

viscosity (40°C, cSt; JIS k2283) of 2,100 to 30,000. Commercial products include, for example, Nissan Polybutene Polyvis 3N, 5N, 10N, 30N (manufactured by NOF Corporation), Nisseki Polybutene HV-35, HV-50, HV-100 (manufactured by Nippon Oil Corporation), Idemitsu Polybutene 35R, 100R (manufactured by Idemitsu Kosan Co., Ltd.), and the like. Addition of polybutene can improve both the coatability and adhesiveness of the adhesive preparation.

The above-mentioned liquid paraffin is a mixture of liquid saturated hydrocarbons obtained from petroleum etc. The liquid paraffin preferably has a specific gravity (20/20°C) of 0.860 to 0.890 and a viscosity of 70 to 130 $mm^2/s$ at 37.8°C. Commercial products include, for example, HI-CALL M-352, M-502 (manufactured by Kaneda Co., Ltd.), and liquid paraffin (manufactured by Chuo Kasei Co., Ltd., Union Sekiyu Kogyo Co., Ltd., and Oriental Pharmaceutical Co., Ltd.). Addition of liquid paraffin can improve both the coatability and adhesiveness of the adhesive preparation.

The above-mentioned light liquid paraffin is a mixture of liquid saturated hydrocarbons obtained from petroleum etc. The light liquid paraffin preferably has a specific gravity (20/20°C) of 0.830 to 0.87 an a viscosity of 5 to 40 $mm^2/s$ at 37.8°C. Commercial products include, for example, HI-CALL M-52, M-72, M-172 (manufactured by Kaneda Co., Ltd.) andNAS-4 (manufactured by NOF Corporation). Addition of light liquid paraffin can improve both the coatability and adhesiveness of the adhesive preparation.

The fatty acid ester preferably refers to an ester synthesized from a fatty acid having 12 to 18 carbon atoms and an alcohol. The fatty acid ester includes, for example, isopropyl myristate, isopropyl palmitate, and oleyl oleate.

In the present invention, the adhesive preparation preferably contains 0.1 to 10 parts by mass of an active ingredient, 1 to 50 parts by mass of a transdermal absorption-promoting agent, 10 to 80 parts by mass of an adhesive base, 5 to 60 parts by mass of a tackifier, and 5 to 60 parts by mass of a softening agent, based on 100 parts by mass of a plaster layer. More preferably, the adhesive preparation contains:

(i) 0.1 to 10 parts by mass of an active ingredient, 1 to 50 parts by mass of a transdermal absorption-promoting agent, 10 to 50 parts by mass of a rubber adhesive base, 10 to 60 parts by mass of a tackifier, and 5 to 60 parts by mass of a softening agent,
(ii) 0.1 to 10 parts by mass of an active ingredient, 1 to 50 parts by mass of a transdermal absorption-promoting agent, 20 to 80 parts by mass of an acrylic adhesive base, 5 to 30 parts by mass of a tackifier, and 5 to 60 parts by mass of a softening agent, or
(iii) 0.1 to 10 parts by mass of an active ingredient, 1 to 50 parts by mass of a transdermal absorption-promoting agent, 20 to 80 parts by mass of a silicone adhesive base, 5 to 30 parts by mass of a tackifier, and 5 to 60 parts by mass of a softening agent.
It is particularly preferred that the adhesive preparation contains 0.5 to 5 parts by mass of an active ingredient, 2 to 15 parts by mass of a transdermal absorption-promoting agent, 15 to 80 parts by mass of an adhesive base, 5 to 60 parts by mass of a tackifier, and 10 to 40 parts by mass of a softening agent, based on 100 parts by mass of a plaster layer. Inter alia, it is even more preferred that the adhesive preparation contains:
(iv) 0.5 to 5 parts by mass of an active ingredient, 2 to 15 parts by mass of a transdermal absorption-promoting agent, 15 to 35 parts by mass of a rubber adhesive base, 20 to 60 parts by mass of a tackifier, and 10 to 40 parts by mass of a softening agent,
(v) 0.5 to 5 parts by mass of an active ingredient, 2 to 15 parts by mass of a transdermal absorption-promoting agent, 20 to 80 parts by mass of an acrylic adhesive base, 5 to 30 parts by mass of a tackifier, and 10 to 40 parts by mass of a softening agent, or
(vi) 0.5 to 5 parts by mass of an active ingredient, 2 to 15 parts by mass of a transdermal absorption-promoting agent, 20 to 80 parts by mass of a silicone adhesive base, 5 to 20 parts by mass of a tackifier, and 10 to 40 parts by mass of a softening agent.

In the foregoing description, the "transdermal absorption-promoting agent" means propylene glycol or a combination of propylene glycol and at least one member selected from the group consisting of polyoxyethylene alkyl ether, polyoxyethylene alkyl ether phosphate or a salt thereof, oleyl alcohol, and sorbitan fatty acid ester. The "tackifier" means a hydrogenated terpene resin or a combination of a hydrogenated terpene resin and at least one member selected from the group consisting of a rosin resin, a petroleum resin, and another terpene resin. Accordingly, the respective aspects are specifically described again in the following. The adhesive preparation of the present invention contains propylene glycol in an amount of preferably 1 to 50 parts by mass, more preferably 2 to 15 parts by mass, based on 100 parts by mass of the plaster layer. The combination of propylene glycol and at least one member selected from the group consisting of polyoxyethylene alkyl ether, polyoxyethylene alkyl ether phosphate or a salt thereof, oleyl alcohol, and sorbitan fatty acid ester is contained in an amount of preferably 1 to 50 parts by mass, more preferably 2 to 15 parts by mass, based on 100 parts by mass of the plaster layer.

In the present invention, the bisphosphonic acid derivative or the like as the active ingredient is dissolved in a solubilizer and used in the form of a solution when a plaster is prepared for the production of the adhesive preparation of the present invention. The "solubilizer" in this case, that is, the solubilizer for the active ingredient in the present

invention, is preferably water because almost all of the bisphosphonic acid derivatives, salts thereof and hydrates of either of the bisphosphonic acid derivative or the salt are excellent in water solubility. When a poorly water-soluble bisphosphonic acid derivative or the like is used, an aqueous alkaline solution such as aqueous sodium hydroxide solution etc. may be used as the solubilizer. Accordingly, the bisphosphonic acid derivative or the like in the present invention is preferably used in the form of an aqueous solution thereof or an aqueous alkaline solution thereof. The amount of the solubilizer used cannot be generalized because it depends on the type of the bisphosphonic acid derivative or the like contained in the plaster layer of the adhesive preparation, the solubility thereof , the content thereof in one preparation, the mass ratio of the dissolved drug to the non-dissolved drug in the adhesive preparation, and the type and compounding amount of the base for external use. Usually, the amount of the solubilizer is preferably 1 to 10 parts by mass based on 1 part by mass of the bisphosphonic acid derivative or the like. The content of the solubilizer in the whole of the plaster layer after drying is preferably 0.1 to 5 parts by mass based on 1 part by mass of the bisphosphonic acid derivative or the like.

The mass ratio of the dissolved drug to the non-dissolved drug in the adhesive preparation is not limited, but when a prolonged application period is not necessary, the dissolved drug is preferably used. In consideration of irritation, the ratio of the non-dissolved drug also may be determined from the relationship between the transdermal permeability rate and the application period.

The adhesive preparation of the present invention comprising the bisphosphonic acid derivative, a salt thereof or a hydrate of either of the bisphosphonic acid derivative or the salt exhibits good transdermal permeability, particularly in an acidic range. For this reason and in view of avoidance of skin irritation, the adhesive preparation may be adjusted to be weakly acidic to neutral, preferably weakly acidic, in the part in contact with the skin to which it is applied. Accordingly, the bisphosphonic acid derivative, a salt thereof or a hydrate of either of the bisphosphonic acid derivative or the salt, when formed into a solution, may be also adjusted to a pH of about 4 to 9 by further adding a buffer. It is also possible to employ a method in which the bisphosphonic acid derivative, a salt thereof or a hydrate of either of the bisphosphonic acid derivative or the salt is dissolved in an aqueous alkaline solution and then neutralized with an aqueous acidic solution to prepare a solution of the bisphosphonic acid derivative, a salt thereof or a hydrate of either of the bisphosphonic acid derivative or the salt.

The buffer used herein is not limited as long as it is a pharmaceutically acceptable buffer, and its examples include citric acid, citric anhydride, sodium citrate, tartaric acid, succinic acid, dl-malic acid, fumaric acid, maleic acid, lactic acid, sodium lactate solution, boric acid, borax, acetic acid, glacial acetic acid, sodium acetate, phosphoric acid, sodium hydrogen phosphate, potassium dihydrogen phosphate, sodium dihydrogen phosphate, anhydrous sodium hydrogen phosphate, anhydrous sodium dihydrogen phosphate, benzoic acid, sodium benzoate, primary, secondary or tertiary phosphate (e.g., sodium dihydrogen phosphate, disodium hydrogen phosphate, anhydrous trisodium phosphate, and trisodium phosphate), sodium metaphosphate, ε-aminocaproic acid, sodium hydroxide, sodium carbonate, sodium bicarbonate, ammonium chloride, sodium chloride, benzalkonium chloride, amino acids or salts thereof (e.g., glycine and L-arginine), diethanolamine, diisopropanolamine, monoethanolamine, triethanolamine, triethanolamine hydrochloride, sodium triethanolamine phosphate ester solution, chlorobutanol, glucose, and rose oil. More preferable examples include organic acids such as citric acid, tartaric acid, succinic acid, malic acid, fumaric acid, maleic acid, acetic acid, etc., salts of these organic acids, primary, secondary or tertiary phosphate (e.g., sodium dihydrogen phosphate, disodium hydrogen phosphate, etc.), amino acids such as glycine etc. or salts of the amino acids, etc., and a mixture of any of the foregoing compounds.

For the purpose of increasing the transdermal absorption of the active ingredient and for another purpose, the plaster layer of the adhesive preparation of the present invention may further be compounded with an additional transdermal absorption-promoting agent, an amphiphilic solubilizing aid ingredient, an emulsifying agent, a lotion base, an additive, a skin irritation reducing agent, a preservative, an antioxidant and/or the like.

The transdermal absorption-promoting agent, the amphiphilic solubilizing aid ingredient, or the lotion base includes higher alcohols (lauryl alcohol, isopropanol, isostearyl alcohol, octyl dodecanol, oleyl alcohol or the like), fatty acids (capric acid, adipic acid, sebacic acid, myristic acid, oleic acid or the like), dibasic acid diesters (diethyl sebacate, diisopropyl sebacate, diisopropyl adipate and the like), triacetin, benzyl alcohol, cetyl lactate, and octyldodecyl lactate. The emulsifying agent includes glycerol fatty acid ester, polyoxyethylene sorbitan fatty acid ester, etc.

The additive includes titanium oxide, talc, magnesium alumina hydroxide, etc.

The skin irritation reducing agent includes glycerin, crotamiton, allantoin, antihistaminic agents (diphenhydramine or the like), and anti-inflammatory agent (glycyrrhetinic acid or the like). A steroid agent etc. may also be incorporated into the adhesive preparation.

The preservative and/or the antioxidant is preferably dibutylhydroxytoluene (BHT), or DL-α-tocopherol. Its compounding amount is preferably about 0.01 parts by mass to about 5 parts by mass , based on 1 part by mass of the bisphosphonic acid derivative, a salt thereof or a hydrate of either of the bisphosphonic acid derivative or the salt.

To achieve good dermal transfer of the drug, the adhesive preparation of the present invention is desirably fixed to the skin surface for sure. The adhesive preparation desirably has adhesiveness to the skin so as to withstand

bathing or sweating for achieving good fixation as well as desirably has strong close adhesion to the skin for achieving good transdermal absorption by increasing the adhesion area of the plaster layer to the skin surface. However, too strong adhesiveness to the skin causes physical stimulus upon peeling off the adhesive preparation for removal after use. This is accompaniedbypeel-off of the stratum corneum, thereby resulting in skin irritation. To increase the close adhesion of the plaster layer to the skin and reduce the physical skin irritation, an easily peelable adsorbent may be added to the plaster layer. Examples of the easily peelable adsorbent include a cross-linked acrylic adhesive polymer, an ethylene-acetic acid-vinyl copolymer, an ethylene-ethyl acrylate copolymer and the like. When the close adhesion and adhesiveness of the drug-containing plaster layer to the skin are poor, a drug-free plaster layer having high adhesiveness and close adhesion to the skin may be disposed on the periphery of the drug-containing plaster layer (frame type) to improve the adhesiveness and close adhesion of the adhesive preparation to the skin.

As a method of evaluating the adhesiveness and close adhesion, an adhesion test method is known. In a 180° peel adhesion test for the adhesive preparation of the present invention, the load for peeling off at a constant rate is preferably a weight of 1 g to 500 g, more preferably a weight of 1 g to 200 g.

In a probe tack test, the load for pulling off at a constant rate is preferably a weight of 100 g to 2000 g, more preferably a weight of 280 g to 2000 g.

In addition, the 180° peel adhesion test and the probe tack test follow the methods described in Test Examples 5 and 6 described later.

The adhesive preparation of the present invention can be produced by a general production method. Examples include (1) a solvent method, (2) a heating method (hot melt method) and (3) a calendar method.

In the solvent method, an active ingredient and a base for external use are dissolved in an organic solvent such as a rubber solvent, fatty acid ester or alcohol, or in a solvent such as water. Next, the mixture is applied onto a release film, a support or the like, and the solvent is dried to form a plaster layer. Preferably, the drying process is conducted at about 20°C to about 100°C for about 30 seconds to about 60 minutes.

In the heating method, an active ingredient and a base for external use are melted and mixed at high temperature. Next, the mixture is applied onto a release film, a support or the like, and cooled to form a plaster layer.

In the calendar method, an active ingredient and a base for external use are mixed by a mixer. Next, the mixture is applied with a calendar roll onto a release film, a support or the like to form a plaster layer.

When the viscosity of an active ingredient and a base for external use is relatively low, the active ingredient and the base are mixed by a general mixer, and then the mixture is applied onto a release film or the like.

By these methods, the active ingredient and the base for external use are applied onto a release film, a support or the like, and the formed plaster layer thereon is dried. Then, the upper surface of the plaster layer on the release film is covered with a support, or the upper surface of the plaster layer on the support is covered with a release film, to integrate the release film, the plaster layer and the support. Lastly, the integrated product is cut to prepare the intended adhesive preparation.

The method for producing the adhesive preparation of the present invention is not limited to the methods described above, and the adhesive preparation may be produced by other efficient methods.

In the present invention, the relative standard deviation of the mass of the plaster layer can be used as a measure of good coatability. For example, in a production process in which a plurality of adhesive preparations are obtained by cutting after coating and drying, test samples are selected at random from the plurality of cut adhesive preparations and measured for their mass. The theoretical mass of the support and the release paper is subtracted from the measured mass of each of the adhesive preparations, to determine the mass of the plaster layer. When the arithmetic mean value of the mass of the plaster layer per adhesive preparation is X and the standard deviation is s, the relative standard deviation is expressed as a percentage of the standard deviation s on the basis of the mean value X. A lower standard deviation is indicative of more uniform coating, i.e., better coatability. When the number of test samples is 3 or more, the relative standard deviation can theoretically be determined, but its accuracy increases with an increase in the number of test samples. The number of test samples may be preferably about 10.

In the present invention, the relative standard deviation determined from 10 test samples is preferably 5% or less. In addition , coating uniformity typically declines in the vicinity of the periphery of a coating area. Thus, in the above-mentioned production process of plural adhesive preparations, a cutting area is preferably set within an area about 5 cm inside from each edge of the coating area, as is the case with production of usual adhesive preparations.

In the present invention, one adhesive preparation is preferably about 1 $cm^2$ to about 200 $cm^2$ in size, more preferably about 2 $cm^2$ to about 70 $cm^2$. The adhesive preparation may be any shape, but is preferably square, rectangular, circular or elliptic. The thickness of the plaster layer in the adhesive preparation is preferably about 10 $\mu$m to about 2000 $\mu$m, more preferably about 20 $\mu$m to about 500 $\mu$m, still more preferably about 20 $\mu$m to about 300 $\mu$m, further more preferably about 30 $\mu$m to about 100 $\mu$m, in order to make the plaster layer withstand a long-term application on the skin and to generate little adhesive residue on the skin after removal by peeling. The adhesive preparation may have a drug-containing plaster layer spread on the whole surface of the adhesive preparation or may have a drug-free plaster layer disposed on the periphery of a drug-containing plaster layer (frame type). That is, the size of the drug-containing

plaster layer in one adhesive preparation is equal to, or smaller than, the size of the adhesive preparation. The drug-free plaster layer to be disposed on the periphery should have strong adhesiveness and close adhesion, and may comprise, for example, the adhesive base, the tackifier, etc. described above.

The active ingredient in the present invention, that is, the bisphosphonic acid derivative or the like, is either a dissolved ingredient or a mixture of dissolved and non-dissolved ingredients to be incorporated into the plaster layer. The bisphosphonic acid derivative or the like may be either a dissolved ingredient or a mixture of dissolved and non-dissolved ingredients as long as it can keep stable and safe and can permeate in a persistent and efficient manner through the skin. Specifically, substantially only a dissolved bisphosphonic acid derivative or the like, or a mixture of a dissolved bisphosphonic acid derivative or the like and a non-dissolved bisphosphonic acid derivative or the like, may be incorporated as the active ingredient into the plaster layer.

Generally, the non-dissolved drug is not involved in transdermal absorption, but as the content of the dissolved drug in the plaster increases, the transdermal absorption rate of the drug increases. Accordingly, drug effect can be sustained for a long time by inclusion of both the non-dissolved and dissolved drugs. In other words, the duration of the pharmacological action is limited by the saturation solubility of the drug in the base for external use. Consequently, when a base for external use that poorly dissolved the drug is used, the effect and the effective blood concentration of the drug may not be sufficiently sustained. In this case, to sufficiently sustain the effect and the effective blood concentration of the drug, the dose should be increased, for example by a means of increasing the content of the drug, increasing the thickness of a plaster layer containing the dissolved drug, or increasing the area of a plaster layer in contact with the skin surface. However, these means have many problems in uncomfortable feeling, adhesiveness, skin irritancy, economic efficiency, and the like.

On the other hand, when the transdermal absorption rate is too high due to a large content of the dissolved drug in the plaster layer, a two-layered adhesive plaster layer made up of the plaster layer coated with a styrene-isoprene-styrene block copolymer resin, a silicone resin, an acrylic copolymer resin or the like may be used to decrease the absorption rate and sustain drug effect.

The concentration of the dissolved drug in the plaster layer directly influences the transdermal absorption rate. Its concentration decreases as the dissolved drug gets absorbed through the skin. An excess of the drug exceeding its saturation solubility in the plaster layer used is dispersed therein as a non-dissolved drug. Thus, the content of the dissolved drug in the plaster layer is determined depending on the base for external use used.

On the other hand, the non-dissolved drug serves to supply the plaster layer with the dissolved drug after the dissolved drug is lost to transdermal absorption. As a result, a high transdermal absorption rate and the effective blood concentration of the drug can be sustained for a long time.

According to the present invention, an adhesive preparation comprising substantially only a dissolved bisphosphonic acid derivative or the like as the active ingredient can be prepared by selecting a plaster layer in which the saturation solubility of the active ingredient is high; in this case, if the absorption rate of the active ingredient is too high, the absorption rate can be regulated by forming a two-layered adhesive preparation having the active ingredient-containing plaster layer coated with an active ingredient-free plaster layer. Alternatively, an adhesive preparation comprising, as the active ingredient, a mixture of the dissolved and non-dissolved bisphosphonic acid derivatives or the like can be prepared. In either case, the adhesive preparation of the present invention that shows desired transdermal absorption, shows a stable blood pharmacokinetics pattern, and sustains drug effect well, can be provided.

In the present invention, the "dissolved bisphosphonic acid derivative or the like" means that the bisphosphonic acid derivative or the like is present under a completely dissolved state in the plaster layer. To be more specific, this means that no crystals of the bisphosphonic acid derivative or the like can be observed in the plaster layer by visual observation or under optical microscopy. Additionally, it is preferable that the bisphosphonic acid derivative or the like can be kept under a completely dissolved state in the plaster layer without being precipitated even at a higher concentration. In order that the bisphosphonic acid derivative or the like is kept dissolved even at a higher concentration, an additive may be further used. The additive is not limited as long as it is excellent in compatibility with the base for external use, is capable of sufficiently dissolving the bisphosphonic acid derivative, a salt thereof or a hydrate of either of the bisphosphonic acid derivative or the salt, and is not separated with time from the base for external use. Such an additive can increases the transdermal absorption rate of the drug at an early stage of application as well as sustains an effective blood concentration of the drug. However, the increase in transdermal absorption rate may cause symptoms of skin irritation. It is thus preferable to prepare an adhesive preparation that can sustain a certain transdermal absorption rate by selecting the concentration and amount of the dissolved drug in the plaster layer. It is preferable as well that this adhesive preparation can show a desired AUC value by regulating the application area size and the application period.

The transdermal absorption rate can be evaluated in a transdermal permeation test. The transdermal permeation test is a method of measuring the transdermal permeation of the drug from the adhesive preparation onto the skin of a rat or the like. The result is shown in terms of a transdermal absorption rate and an 8-hour cumulative permeated amount. When the transdermal permeability rate is high, there is a possibility of the onset of skin irritation, and when the rate is low, a prolonged application period is necessary. In the present invention, the cumulative permeated amount is preferably

10 μg/cm$^2$/8 hr or more.

A release test can be used as a test for evaluating the releasability of the active ingredient from the plaster layer. The release test is a test not using a skin unlike the transdermal permeation test using a skin, and involves measuring the amount of the active ingredient which is released from the plaster layer to an aqueous system. The amount of the released active ingredient measured in this test is preferably about 40%/8 hr to about 100%/8 hr, which can be considered good in releasability of the active ingredient.

The ratio of the amount of the active ingredient absorbed transdermally to the active ingredient contained in the plaster layer is referred to as availability.

This availability (E%) is determined conveniently by measuring the residual ratio (R%) of the active ingredient in the plaster layer after application to the skin and then subtracting the residual ratio from 100%. That is, the availability (E) is determined using the following equation:

$$E = 100 - R = 100 - (C_1/C_0) \times 100,$$

wherein $C_0$ is the compounding amount of the active ingredient per unit area of the plaster layer before application to the skin, and $C_1$ is the remaining amount of the active ingredient per unit area of the plaster layer after application to the skin.

Generally, the oral absorption rate (availability in oral administration) of the bisphosphonic acid derivative or the like is as low as about 3% or less. It is said that the availability of general adhesive preparations marketed at present is about 20%. In the present invention, the availability is preferably about 20% or more, and the availability of about 20% or more can be considered good in availability.

In the present invention, the "mixture of the dissolved and non-dissolved bisphosphonic acid derivatives or the like" means that the dissolved bisphosphonic acid derivative or the like and the non-dissolved bisphosphonic acid derivative or the like, specifically the bisphosphonic acid derivative or the like in a dissolved state and the bisphosphonic acid derivative or the like in a crystalline or noncrystalline state (solid), are present as a mixture in the plaster layer. To supply the plaster layer with the dissolved bisphosphonic acid derivative or the like after the dissolved one is lost to rapid absorption, re-dissolution of the non-dissolved bisphosphonic acid derivative or the like occurs rapidly. Thus, drug effect can be sustained via the absorption of the re-dissolved bisphosphonic acid derivative or the like. The ratio of the dissolution rate of the non-dissolved bisphosphonic acid derivative or the like in the plaster to the absorption rate of the whole of the bisphosphonic acid derivative or the like in the plaster is preferably about 0.1 or more. When this ratio is low, drug effect is not sustained well because re-dissolution of the non-dissolved bisphosphonic acid derivative or the like is insufficient relative to the reduction of the dissolved drug.

It is particularly preferred that the ratio of the dissolved bisphosphonic acid derivative or the like to the non-dissolved bisphosphonic acid derivative or the like (dissolved type : non-dissolved type) is adjusted to the range of from 1 : 0.01 to 1 : 10. This can further enhance the action of the transdermal absorption-promoting agent on conversion of the non-dissolved bisphosphonic acid derivative or the like into the dissolved one, can increase the persistent transdermal absorption of the bisphosphonic acid derivative or the like, and can show the therapeutic effect at an early stage. This ratio can be regulated by changing the compounding ratio of the bisphosphonic acid derivative or the like to the base for external use or the like.

The adhesive preparation of the present invention is extremely excellent in patients' compliance with medication, has no irritancy to gastrointestinal mucosa, has no restriction on the administration unlike an oral preparation, causes no pain unlike an injection, and finds no need for long intravenous drip infusion. The adhesive preparation of the present invention is further characterized by being excellent in transdermal permeability, being very slightly skin irritant, and particularly showing a high AUC value. Thus, the adhesive preparation of the present invention shows a preferable AUC value by single application even in patients with diseases (for example, osteoporosis) requiring a long-term medication. Such an adhesive preparation does not always have to be administered daily, but can be administered intermittently to the above patients. Accordingly, the adhesive preparation of the present invention can immensely reduce burden on patients.

The adhesive preparation of the present invention exhibits its effect by binding of the bisphosphonic acid derivative to the bone or calcium. Such an adhesive preparation is useful as a preventive and/or therapeutic preparation for osteopenia and osteoporosis such as postmenopausal osteoporosis, steroid-induced osteoporosis and senile osteoporosis, and bone diseases such as hypercalcemia accompanying malignant tumors, etc. , multiple myeloma, bone metastasis from cancer, rheumatoid arthritis, spondylosis deformans, osteoarthritis, periodontal diseases and Paget's disease.

The adhesive preparation of the present invention can circumvent the following problems resulting from the characteristics of the bisphosphonic acid derivative or the like : strict adherence to conditions for the administration like

an oral preparation; gastrointestinal disorders in the esophagus, gastric mucosa, etc.; pain like an injection; and adverse drug reactions (for example, renal damage, hypocalcemia, jawbone necrosis and the like) caused by a temporarily high blood concentration of the drug like an oral preparation to be intermittently administered in a large single dose or an injection. Because the active ingredient can be absorbed persistently through the skin, the adhesive preparation has advantages that patients' compliance with medication is much more excellent as compared with an oral preparation and an injection and that the application period can be selected as necessary. Consequently, the adhesive preparation of the present invention can improve the quality of life of the elderly and patients having difficulty in oral administration and is significantly useful as a long-term preventive and/or therapeutic preparation for bone diseases such as osteoporosis.

One aspect of the invention relates to use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent as an adhesive preparation; and use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin , anadhesivebase, and a softening agent as an adhesive preparation for prevention and/or treatment of bone diseases. The adhesive preparation using such a composition and its preferable aspect are the same adhesive preparation as described above. The adhesive preparation produced by using such a composition is useful as a preventive and/or therapeutic preparation for the bone diseases described above.

Another aspect of the invention relates to use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent for the production of an adhesive preparation; and use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent for the production of an adhesive preparation for prevention and/or treatment of bone diseases. The method of producing the adhesive preparation by using such a composition and its preferable aspect are the same as described above.

Another aspect of the invention relates to a method of applying, to the skin, a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent; and a method of preventing and/or treating bone diseases, which comprises applying, to the skin, a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent. The method of applying such a composition is preferably a method which comprises producing the adhesive preparation described above by using the composition and then applying the adhesive preparation to the skin. The adhesive preparation, the method for producing the same, and its preferable aspect are the same as described above.

## Examples

Hereinafter, the present invention will be explained by reference to the following examples, but the scope of the present invention is not limited by these examples.

Unless otherwise noted, the coating area of the plaster was roughly rectangular in the size of about 30x50 cm. The adhesive preparation was prepared for use in experiments by cutting within an area about 5 cm or more inside from each edge of the coating area as long as the cutting gave as many sheets of the adhesive preparation as possible. Unless otherwise noted, the terms "parts" and "%" mean "parts by mass" and "% by mass", respectively.

### Example 1

Ethyl acetate (100 g) was added to a styrene-isoprene-styrene block copolymer (30 g, KRATON D-1107 (trade name) manufactured by Kraton JSR Elastomers K.K.), a hydrogenated terpene resin (30 g, CLEARON M-115, manufactured by Yasuhara Chemical Co., Ltd.), light liquid paraffin (25 g, HI-CALL M-72 (trade name) manufactured by Kaneda Co., Ltd.), polybutene (10 g, Polyvis (trade name) manufactured by NOF Corporation), propylene glycol (3 g, manufactured by Asahi Glass Co., Ltd.), and polyoxyethylene behenyl ether (1 g, BB-10 (trade name) manufactured by Nikko Chemicals Co., Ltd.). After the mixture was uniformly dissolved, it was mixed with a solution of minodronic acid hydrate (1 g; CAS No. 155648-60-5) in 5% aqueous sodium hydroxide solution (7 g), to prepare a plaster. The plaster was applied onto a release film (polyester film; Teijin Purex (trade name) manufactured by Teijin DuPont Films) such

that the thickness of the plaster after drying could be about 50 μm. Following this, the plaster was dried with hot air at about 75°C for 4 minutes. The surface of the dried plaster was covered with a support film (polyester film; Teijin NS (trade name) manufactured by Teijin DuPont Films). The resulting product was cut into a square shape with four curved corners, sized 3.2×3.2 cm (10 $cm^2$), to prepare an adhesive preparation (active ingredient content: 0.5 mg/10 $cm^2$).

Example 2

Ethyl acetate (10 g) was added to a styrene-isoprene-styrene block copolymer (2.5 g, KRATON D-1107 (trade name) manufactured by Kraton JSR Elastomers K.K.), a hydrogenated terpene resin (3.0 g, CLEARON M-115 (trade name) manufactured by Yasuhara Chemical Co. , Ltd.), glycerol ester of hydrogenated rosin (0.5 g, KE-311 (trade name) manufactured by Arakawa Chemical Industries, Ltd.), liquid paraffin (2.5 g, HI-CALL M-352 manufactured by Kaneda Co. , Ltd.), polybutene (1.0 g, Polyvis (trade name) manufactured by NOF Corporation), propylene glycol (0.3 g, manufactured by Asahi Glass Co., Ltd.), and polyoxyethylene behenyl ether (0.1 g, BB-10 (trade name) manufactured by Nikko Chemicals Co., Ltd.). After the mixture was uniformly dissolved, it was mixed with a solution of minodronic acid hydrate (0.1 g) in 5% aqueous sodium hydroxide solution (0.7 g), to prepare a plaster. The plaster was applied onto a release film (polyester film; Teijin Purex (trade name) manufactured by Teijin DuPont Films) such that the thickness of the plaster after drying could be about 50 μm. Following this, the plaster was dried with hot air at about 100°C for 20 minutes. The surface of the dried plaster was covered with a support film (polyester film; Teijin NS (trade name) manufactured by Teijin DuPont Films). The resulting product was cut into a square shape with four curved corners, sized 3.2×3.2 cm (10 $cm^2$), to prepare an adhesive preparation (active ingredient content: 0.5 mg/10 $cm^2$).

Examples 3(1) to 3(3)

A styrene-isoprene-styrene block copolymer (KRATON D-1107 (trade name) manufactured by Kraton JSR Elastomers K.K.), a hydrogenated terpene resin (CLEARON M-115 (trade name) manufactured by Yasuhara Chemical Co., Ltd.), glycerol ester of hydrogenated rosin (KE-3111 (trade name) manufactured by Arakawa Chemical Industries, Ltd.), liquid paraffin (HI-CALL M-352 (trade name) manufactured by Kaneda Co., Ltd.), polybutene (Polyvis (trade name) manufactured by NOF Corporation), propylene glycol (manufactured by Asahi Glass Co., Ltd.), polyoxyethylene behenyl ether (BB-10 (trade name) manufactured by Nikko Chemicals Co., Ltd.), isopropyl myristate, and minodronic acid hydrate in the respective amounts shown in Table 1 were subjected to the same procedure as in Example 1. Then, the resulting product was cut into a square shape with four curved corners, sized 3.2×3.2 cm (10 $cm^2$), to prepare an adhesive preparation (active ingredient content: 0.5 mg/10 $cm^2$).

Table 1

| Example | 3(1) | 3(2) | 3(3) |
|---|---|---|---|
| Styrene-isoprene-styrene block copolymer | 15 | 20 | 20 |
| Hydrogenated terpene resin | 35 | 35 | 35 |
| Glycerol ester of hydrogenated rosin | 10 | 12.5 | 12.5 |
| Liquid paraffin | 25 | 17.5 | 12.5 |
| Polybutene | 10 | 10 | 10 |
| Propylene glycol | 3 | 3 | 3 |
| Polyoxyethylene behenyl ether | 1 | 1 | 1 |
| Isopropyl myristate | - | - | 5 |
| Minodronic acid hydrate | 1 | 1 | 1 |
| unit:g | | | |

Example 4

Ethyl acetate (100 g) was added to a styrene-isoprene-styrene block copolymer (25 g, KRATON D-1107 (trade name) manufactured by Kraton JSR Elastomers K.K.), a hydrogenated terpene resin (35 g, CLEARON M-115 (trade name) manufactured by Yasuhara Chemical Co., Ltd.), liquid paraffin (34 g, HI-CALL M-352 (trade name) manufactured by Kaneda Co., Ltd.), propylene glycol (3 g, manufactured by Asahi Glass Co., Ltd.), tripolyoxyethylene cetyl ether

phosphate (1.2 g, TCP-5 (trade name) manufactured by Nikko Chemicals Co., Ltd.), and sorbitan sesquioleate (0.8 g, SO-15EX (trade name) manufactured by Nikko Chemicals Co. , Ltd.). After the mixture was uniformly dissolved, it was mixed with a solution of minodronic acid hydrate (1 g) in 5% aqueous sodium hydroxide solution (7 g), to prepare aplaster. The plaster was applied onto a release film (polyester film; Teijin Purex (trade name) manufactured by Teijin DuPont Films) such that the thickness of the plaster after drying could be about 50 μm. Following this, the plaster was dried with hot air at about 75°C for 4 minutes. The surface of the dried plaster was covered with a support film (polyester film; Teijin NS (trade name) manufactured by Teijin DuPont Films). The resulting product was cut into a square shape with four curved corners, sized 3.2×3.2 cm (10 $cm^2$), to prepare an adhesive preparation (active ingredient content: 0.5 mg/10 $cm^2$).

Example 5

Ethyl acetate (100 g) was added to a styrene-isoprene-styrene block copolymer (25 g, KRATON D-1107 (trade name) manufactured by Kraton JSR Elastomers K.K.), a hydrogenated terpene resin (35 g, CLEARON M-115 (trade name) manufactured by Yasuhara Chemical Co., Ltd.), liquid paraffin (33 g, HI-CALL M-352 (trade name) manufactured by Kaneda Co., Ltd.), propylene glycol (3 g, manufactured by Asahi Glass Co., Ltd.), tripolyoxyethylene cetyl ether phosphate (1.2 g, TCP-5 (trade name) manufactured by Nikko Chemicals Co., Ltd.), and sorbitan sesquioleate (0.8 g, SO-15EX (trade name) manufactured by Nikko Chemicals Co. , Ltd.). After the mixture was uniformly dissolved, it was mixed with a solution of minodronic acidhydrate (2 g) in 5% aqueous sodium hydroxide solution (14 g), to prepare aplaster. The plaster was applied onto a release film (polyester film; Teijin Purex (trade name) manufactured by Teijin DuPont Films) such that the thickness of the plaster after drying could be about 50 μm. Following this, the plaster was dried with hot air at about 75°C for 4 minutes. The surface of the dried plaster was covered with a support film (polyester film; Teijin NS (trade name) manufactured by Teijin DuPont Films). The resulting product was cut into a square shape with four curved corners, sized 3.2×3.2 cm (10 $cm^2$), to prepare an adhesive preparation (active ingredient content: 1 mg/10 $cm^2$).

Example 6

Ethyl acetate (100 g) was added to a styrene-isoprene-styrene block copolymer (30 g, KRATON D-1107 (trade name) manufactured by Kraton JSR Elastomers K.K.), a hydrogenated terpene resin (30 g, CLEARON M-115 (trade name) manufactured by Yasuhara Chemical Co., Ltd.), light liquid paraffin (25 g, HI-CALL M-72 (trade name) manufactured by Kaneda Co. , Ltd.), polybutene (10 g, Polyvis (trade name) manufactured by NOF Corporation), propylene glycol (3g, manufactured by Asahi Glass Co., Ltd.), and polyoxyethylene behenyl ether (1 g, BB-10 manufactured by Nikko Chemicals Co. , Ltd.). After the mixture was uniformly dissolved, it was mixed with a solution of minodronic acid hydrate (0.4 g) in 5% aqueous sodium hydroxide solution (2.8 g), to prepare a plaster. The plaster was applied onto a release film (polyester film; Teijin Purex (trade name) manufactured by Teijin DuPont Films) such that the thickness of the plaster after drying could be about 50 μm. Following this, the plaster was dried with hot air at about 75°C for 4 minutes. The surface of the dried plaster was covered with a support film (polyester film; Teijin NS (trade name) manufactured by Teijin DuPont Films). The resulting product was cut into a square shape with four curved corners, sized 3.2×3.2 cm (10 $cm^2$), to prepare an adhesive preparation (active ingredient content: 0.2 mg/10 $cm^2$).

Examples 7(1) to 7(4)

A styrene-isoprene-styrene block copolymer (2.5 g, KRATON D-1107 (trade name) manufactured by Kraton JSR Elastomers K.K.), a hydrogenated terpene resin (3.0 g, CLEARON M-115 (trade name) manufactured by Yasuhara Chemical Co., Ltd.), glycerol ester of hydrogenated rosin (0.5 g, KE-311 (trade name) manufactured by Arakawa Chemical Industries, Ltd.), liquid paraffin (2.5 g, HI-CALL M-352 (trade name) manufactured by Kaneda Co., Ltd.), polybutene (1.0 g, Polyvis (trade name) manufactured by NOF Corporation), propylene glycol (0.3 g, manufactured by Asahi Glass Co., Ltd.), polyoxyethylene behenyl ether (0.1 g, BB-10 (trade name) manufactured by Nikko Chemicals Co., Ltd.), and each of the bisphosphonic acid derivatives and the like (0.1 g) shown in Table 2 were subjected to the same procedure as in Example 2. Then, the resulting product was cut into a square shape with four curved corners, sized 3.2×3.2 cm (10 $cm^2$), to prepare an adhesive preparation (active ingredient content: 0.5 mg/10 $cm^2$).

Table 2

| Example | Bisphosphonic acid derivative, etc. (CAS No.) |
|---|---|
| 7(1) | Zoledronate disodium (165800-07-7) |
| 7(2) | Ibandronate sodium hydrate (138926-19-9) |

(continued)

| Example | Bisphosphonic acid derivative, etc. (CAS No.) |
|---|---|
| 7(3) | Risedronate sodium hydrate (115436-72-1) |
| 7(4) | Alendronate sodium hydrate (121268-17-5) |

Example 8

An appropriate amount of ethyl acetate was added to a silicone adhesive agent (solid content 7.5 g, MD7-4502 (trade name) manufactured by Dow Corning Corporation), a hydrogenated terpene resin (0.5 g, M-115 (trade name) manufactured by Yasuhara Chemical Co., Ltd.), liquidparaffin (1.5 g, HI-CALLM-352 (trade name) manufactured by Kaneda Co., Ltd.), propylene glycol (0.3 g, manufactured by Asahi Glass Co., Ltd.), and polyoxyethylene behenyl ether (0.1 g, BB-10 manufactured by Nikko Chemicals Co., Ltd.). After the mixture was uniformly dissolved, it was mixed uniformly with a solution of minodronic acid hydrate (0.1 g) in 5% aqueous sodium hydroxide solution (0.7 g), to prepare a plaster. The plaster was applied onto a release film (polyester film; Teijin Purex (trade name) manufactured by Teijin DuPont Films) such that the thickness of the plaster after drying could be about 50 μm. Following this, the plaster was dried with hot air at about 100°C for 20 minutes. The surface of the dried plaster was covered with a support film (polyester film; Teijin NS (trade name) manufactured by Teijin DuPont Films). The resulting product was cut into a square shape with four curved corners, sized 3.2x3.2 cm (10 $cm^2$), to prepare an adhesive preparation (active ingredient content: 0.5 mg/10 $cm^2$).

Examples 9(1) to 9(4)

A silicone adhesive agent (solid content 7.5 g, MD7-4502 (trade name) manufactured by Dow Corning Corporation), a hydrogenated terpene resin (0.5 g, M-115 (trade name) manufactured by Yasuhara Chemical Co., Ltd.), liquid paraffin (1.5 g, HI-CALL M-352 (trade name) manufactured by Kaneda Co. , Ltd.), propylene glycol (0.3 g, manufactured by Asahi Glass Co. , Ltd.), polyoxyethylene behenyl ether (0.1 g, BB-10 (trade name) manufactured by Nikko Chemicals Co., Ltd.), and each of the bisphosphonic acid derivatives and the like (0.1 g) shown in Table 3 were subjected to the same procedure as in Example 7. Then, the resulting product was cut into a square shape with four curved corners, sized 3.2x3.2 cm (10 $cm^2$), to prepare an adhesive preparation (active ingredient content: 0.5 mg/10 $cm^2$).

Table 3

| Example | Bisphosphonic acid derivative, etc. (CAS No.) |
|---|---|
| 9(1) | Zoledronate disodium (165800-07-7) |
| 9(2) | Ibandronate sodium hydrate (138926-19-9) |
| 9(3) | Risedronate sodium hydrate (115436-72-1) |
| 9(4) | Alendronate sodium hydrate (121268-17-5) |

Example 10

A methacrylic acid-(n-butyl acrylate) copolymer (solid content 6.9 g, Ultrasol Q-1 (trade name) manufactured by Ganz Chemical Co., Ltd.), a hydrogenated terpene resin (0.5 g, M-115 (trade name) manufactured by Yasuhara Chemical Co., Ltd.), liquid paraffin (1.0 g, HI-CALL M-352 (trade name) manufactured by Kaneda Co., Ltd.), polybutene (1.0 g, Polyvis (trade name) manufactured by NOF Corporation), propylene glycol (0.3 g, manufactured by Asahi Glass Co., Ltd.), polyoxyethylene behenyl ether (0.1 g, BB-10 (trade name) manufactured by Nikko Chemicals Co., Ltd.), sorbitan fatty acid ester (0.05 g, SO-10 (trade name) manufactured by Nikko Chemicals Co., Ltd.), polyoxyethylene sorbitan fatty acid ester (0.05 g, TO-10 (trade name) manufactured by Nikko Chemicals Co., Ltd.) were added, uniformly dissolved, and uniformly mixed with a solution of minodronic acid hydrate (0.1 g) in 5% aqueous sodium hydroxide solution (0.7 g), to prepare a plaster. The plaster was applied onto a release film (polyester film; Teijin Purex (trade name) manufactured by Teijin DuPont Films) such that the thickness of the plaster after drying could be about 50 μm. Following this, the plaster was dried with hot air at about 100°C for 20 minutes. The surface of the dried plaster was covered with a support film (polyester film; Teijin NS (trade name) manufactured by Teijin DuPont Films). The resulting product was cut into a square shape with four curved corners, sized 3.2×3.2 cm (10 $cm^2$), to prepare an adhesive preparation (active ingredient content: 0.5 mg/10 $cm^2$).

Examples 11(1) to 11(4)

A methacrylic acid-(n-butyl acrylate) copolymer (solid content 6.9 g, Ultrasol Q-1 (trade name) manufactured by Ganz Chemical Co., Ltd.), a hydrogenated terpene resin (0.5 g, M-115 (trade name) manufactured by Yasuhara Chemical Co., Ltd.), liquid paraffin (1.0 g, HI-CALL M-352 (trade name) manufactured by Kaneda Co., Ltd.), polybutene (1.0 g, Polyvis (trade name) manufactured by NOF Corporation), propylene glycol (0.3 g, manufactured by Asahi Glass Co., Ltd.), polyoxyethylene behenyl ether (0.1 g, BB-10 (trade name) manufactured by Nikko Chemicals Co., Ltd.), sorbitan fatty acid ester (0.05 g, SO-10 (trade name) manufactured by Nikko Chemicals Co., Ltd.), polyoxyethylene sorbitan fatty acid ester (0.05 g, TO-10 (trade name) manufactured by Nikko Chemicals Co., Ltd.) and each of the bisphosphonic acid derivatives and the like (0.1 g) shown in Table 4 were subjected to the same procedure as in Example 9. Then, the resulting product was cut into a square shape with four curved corners, sized 3.2×3.2 cm (10 $cm^2$), to prepare an adhesive preparation (active ingredient content: 0.5 mg/10 $cm^2$).

Table 4

| Example | Bisphosphonic acid derivative, etc. (CAS No.) |
|---|---|
| 11(1) | Zoledronate disodium (165800-07-7) |
| 11(2) | Ibandronate sodium hydrate (138926-19-9) |
| 11(3) | Risedronate sodium hydrate (115436-72-1) |
| 11(4) | Alendronate sodium hydrate (121268-17-5) |

Comparative Example 1

In a laboratory scale, the following adhesive preparation was prepared for trial. To be specific, ethyl acetate (10 g) was added to a styrene-isoprene-styrene block copolymer (4.0 g; 40.0 parts), a terpene resin (3.45 g; 34.5 parts), an aliphatic hydrocarbon resin (1.0 g; 10.0 parts), N-methyl-2-pyrrolidone (0.5 g; 5.0 parts), α-monoisostearyl glyceryl ether (0.25 g; 2.5 parts), isopropyl myristate (0.5 g; 5.0 parts), and sorbitan fatty acid ester (0.1 g; 1.0 part). After the mixture was dissolved uniformly, this solution was mixed uniformly with a uniform mixture prepared by dissolving minodronic acid hydrate (0.2 g: 2.0 parts) in 2 M aqueous sodium hydroxide solution (0.62 g) and adding water (0.18 g) thereto, to produce a plaster. The plaster was applied onto a release film (polyester film; Teijin Purex (trade name) manufactured by Teijin DuPont Films) such that the thickness of the plaster after drying could be about 100 μm. Following this, the plaster was dried with hot air at about 100°C for 20 minutes. The surface of the dried plaster was covered with a support film (polyester film; Teij in NS (trade name) manufactured by Teijin DuPont Films). The resulting product was cut into a square shape with four curved corners, sized 3.2x3.2 cm (10 $cm^2$), to prepare an adhesive preparation (active ingredient content: 2 mg/10 $cm^2$).

Comparative Example 2

In a bench scale in which the above-mentioned materials were used in larger amounts than in the laboratory scale in Comparative Example 1, an adhesive preparation was produced for trial in the following manner. Ethyl acetate (100 g) was added to a styrene-isoprene-styrene block copolymer (40 g; 40.0 parts), a terpene resin (34.5 g; 34.5 parts), an aliphatic hydrocarbon resin (10 g; 10.0 parts), N-methyl-2-pyrrolidone (5 g; 5.0 parts), α-monoisostearyl glyceryl ether (2.5 g; 2.5 parts), isopropyl myristate (5 g; 5.0 parts), and sorbitan fatty acid ester (1 g; 1. 0 part). Then, the mixture was dissolved uniformly. When this solution was mixed uniformly with a uniform mixture prepared by dissolving minodronic acid hydrate (2.0 g: 2.0 parts) in 2 M aqueous sodium hydroxide solution (6.2 g) and adding water (1.8 g) thereto, the amounts of the materials to be treated were so large that lengthy stirring and mixing were required. The plaster obtained by stirring and mixing was a semisolid viscous material. This was assumed to be attributable to the lengthy mixing and stirring of the aqueous sodium hydroxide solution, the styrene-isoprene-styrene block copolymer, etc. in this formulation. Although this plaster was attempted to be applied onto a release film (polyester film; Teijin Purex (trade name) manufactured by Teijin DuPont Films) such that the thickness of the plaster after drying could be about 100 μm, the coating surface became not uniform, but wavy. Thus, an adhesive preparation failed to be produced.

Comparative Example 3

Ethyl acetate (10 mL) was added to a styrene-isoprene-styrene block copolymer (4.0 g; 40.0 parts), a terpene resin (3.45 g; 34.5 parts), an aliphatic hydrocarbon resin (1.5 g; 15.0 parts), α-monoisostearyl glyceryl ether (0.25 g; 2.5

parts), isopropyl myristate (0.5 g; 5.0 parts), and sorbitan fatty acid ester (0.1 g; 1.0 part). After the mixture was dissolved uniformly, this solution was mixed uniformly with a uniform mixture prepared by dissolving minodronic acid hydrate (0.2 g: 2.0 parts) in 2 M aqueous sodium hydroxide solution (0.62 g) and adding water (0.18 g) thereto, to produce a plaster. The plaster was applied onto a release film (polyester film; Teijin Purex (trade name) manufactured by Teijin DuPont Films) such that the thickness of the plaster after drying could be about 100 μm. Following this, the plaster was dried with hot air at about 100°C for 20 minutes. The surface of the dried plaster was covered with a support film (polyester film/vinylon nonwoven fabric laminate, manufactured by OG Corporation). The resulting product was cut into a square shape with four curved corners, sized 3.2×3.2 cm (10 $cm^2$), to prepare an adhesive preparation (active ingredient content: 2 mg/10 $cm^2$).

Comparative Example 4

Ethyl acetate (100 g) was added to a styrene-isoprene-styrene block copolymer (30 g, KRATON D-1107 (trade name) manufactured by Kraton JSR Elastomers K.K.), a hydrogenated terpene resin (30 g, CLEARON M-115 (trade name) manufactured by Yasuhara Chemical Co., Ltd.), light liquid paraffin (25 g, HI-CALL M-72 (trade name) manufactured by Kaneda Co., Ltd.), polybutene (10 g, Polyvis (trade name) manufactured by NOF Corporation), propylene glycol (3 g, manufactured by Asahi Glass Co., Ltd.), and polyoxyethylene behenyl ether (1 g, BB-10 (trade name) manufactured by Nikko Chemicals Co. , Ltd.). Then, the mixture was uniformly dissolved, to prepare a plaster. The plaster was applied onto a release film (polyester film; Teijin Purex (trade name) manufactured by Teijin DuPont Films) such that the thickness of the plaster after drying could be about 50 μm. Following this, the plaster was dried with hot air at about 75°C for 4 minutes. The surface of the dried plaster was covered with a support film (polyester film; Teijin NS (trade name) manufactured by Teijin DuPont Films). The resulting product was cut into a square shape with four curved corners, sized 3.2x3.2 cm (10 $cm^2$), to prepare an adhesive preparation.

Some of the adhesive preparations obtained in the Examples and Comparative Examples were tested as follows.

Test Example 1: *In Vitro* Transdermal Absorption Test

Pieces of the hairless abdominal skin were isolated from of a male Wistar/ST rat (7 weeks of age). The skin piece was mounted with the dermis side facing down in a two-chamber diffusion cell. Each test preparation was placed on the epidermis side of the skin, and a phosphate buffer, pH 7.4, was poured into the receiver side of the cell. The temperature was kept constant by circulating water at 32°C around the cell. The phosphate buffer (1000 μL) was periodically collected from the cell, and immediately the same volume of buffer was added to the cell for replenishment. The concentration of minodronic acid was measured over a period of 8 hours by HPLC under the following conditions, and the cumulative permeated amount thereof in terms of hydrate was determined. The results are shown in Table 5 below.

<HPLC Conditions>

Column: Develosil ODS-5-HG (manufactured by Nomura Chemical Co., Ltd.)

Column temperature: 35°C

Mobile phase: 4.46 g of pyrophosphoric acid·decahydrate was dissolved in about 950 mL of water. To this was added 2.5 mL of a 25% solution of hexadecyl trimethyl ammonium hydroxide in methanol, followed by water to adjust the total volume to 1000 mL. The pH was adjusted to 7.5 with phosphoric acid. Then, 150 mL of acetonitrile was added to 850 mL of the resulting solution to prepare the mobile phase.

Detection wavelength: excitation wavelength 230 nm, absorption wavelength 397 nm.

Injection volume: 5 μL

Retention time: about 10 min.

Table 5

| | Cumulative permeated amount for 8 hours (μg/cm²) | Content of active ingredient (μg/cm²) | Permeability (%) (Cumulative permeated amount / content of active ingredient x 100) |
|---|---|---|---|
| Example 1 | 27.14 | 50 | 54.3% |
| Example 4 | 11.24 | 50 | 22.5% |
| Example 5 | 41.04 | 100 | 41.0% |
| Example 6 | 13.35 | 20 | 66.8% |
| Comparative example 1 | 47.00 | 200 | 23.5% |
| Comparative example 3 | 0.93 | 200 | 0.5% |

As a result, the adhesive preparation in Example 5 showed the almost same cumulative permeated amount for 8 hours as the adhesive preparation in Comparative Example 1 containing the 2-fold amount of the active ingredient. The adhesive preparations in Examples 1 and 4 each contain the 1/4 amount of the active ingredient relative to the comparative preparation, and the adhesive preparation in Example 6 contains the 1/10 amount of the active ingredient relative to the comparative preparation. Even these preparations showed almost the same or higher permeability (%) (determined by dividing the cumulative permeated amount by the content of the active ingredient) as compared with the preparation in Comparative Example 1. Accordingly, the adhesive preparations in these examples are extremely superior in the permeability to the preparation in Comparative Example 1.

Test Example 2: *In Vivo* Transdermal Absorption Test (1)

The abdominal skin of each of male hairless rats (10 weeks of age, HWY/Slc, n = 6) was shaved using an electric hair clipper. Each of the adhesive preparations prepared in the Examples or Comparative Examples was applied to the abdominal part and then application site was blocked up for 48 hours by wrapping with a nonwoven fabric adhesive bandage (Meshpore, manufactured by Nichiban Co. , Ltd.). The adhesive preparation was removed after 48 hours of blockage. 3, 8, 24, 32 and 48 hours after the adhesive preparation was applied and 6, 24, 48, 72 and 120 hours after the adhesive preparation was removed, blood (about 0.3 mL) was collected from jugular vein without anesthesia using a heparinized polypropylene disposable syringe. The blood plasma was transferred to a polypropylene container. The thus obtained plasma was immediately ice-cooled and then cryopreserved. Thereafter, blood concentration of the active ingredient (ng/mL) at each time was measured, and the AUC value for a period up to 120 hours after removal was determined. The average of the AUC values from 6 rats was calculated. The results are shown in Table 6 below.

The blood concentration was measured by the following HPLC method, and the quantitation limit was 0.05 ng/mL.

<Method of Measuring Blood Concentration of Minodronic Acid>

Plasma pretreatment method: After addition of an internal standard solution, each plasma sample was deproteinized with 6% perchloric acid and centrifuged. Then, to the supernatant was added an aqueous calcium phosphate/hydrochloric acid solution and then an aqueous sodium hydroxide solution. The resulting mixture was centrifuged again, to collect precipitate. The precipitate was dissolved with an aqueous sodium hydroxide/EDTA solution to give a sample for HPLC assay.

<HPLC Conditions>

Column: Develosil ODS-UG-5 (manufactured by Nomura Chemical Co., Ltd.)
Column temperature: 40°C
Mobile phase: 10 mmol/L sodium diphosphate solution, pH 7, containing 1 mmol/L tetrabutyl ammonium phosphate : acetonitrile (95 : 5)
Flow rate: 1.0 mL/min
Detection wavelength: fluorescence excitation wavelength 281 nm, fluorescence wavelength 391 nm.

Table 6

| | AUC value for a period up to 120 hours after removal (ng·h/mL) |
|---|---|
| Example 1 | 658.01 |
| Example 5 | 211.55 |

As a result, the adhesive preparations of the present invention (Examples 1 and 5) exhibited persistent absorption of the active ingredient into blood and showed a high AUC value during 48-hour application period.

Test Example 3: *In Vivo* Transdermal Absorption Test (2)

The abdominal skin of each of male hairless rats (10 weeks of age, HWY/Slc, n = 8) was shaved using an electric hair clipper. Each of the adhesive preparations prepared in Example 1 or 6 was applied to the abdominal part and then the application site was blocked up for 7 days by wrapping with a nonwoven fabric adhesive bandage (Meshpore, manufactured by Nichiban Co., Ltd.). The adhesive preparation was removed after 7 days of blockage. 3 hours, 8 hours, 24 hours, 2 days, 3 days, 4 days, 5 days and 7 days after the adhesive preparation was applied, and 6 hours, 24 hours, 2 days, 3 days, 4 days, 5 days and 7 days after the adhesive preparation was removed, blood (about 0.3 mL) was collected from jugular vein without anesthesia using a heparinized polypropylene disposable syringe. The blood plasma was transferred to a polypropylene container. The animals were divided into two groups (Animal Nos. 1 to 4 and Animal Nos. 5 to 8). Blood was drawn from each animal in one group (n = 4) at every other blood collection point. At the remaining intervening blood collection points, blood was drawn from each animal in the other group (n = 4). The thus obtained plasma was immediately ice-cooled and then cryopreserved. Thereafter, blood concentration of the active ingredient (ng/mL) at each time was measured, and the AUC value for 7 days from application (during 7-day blocking period) and the AUC value for 14 days from application to day 7 after removal were determined. Each average of the AUC values from 4 rats was calculated. The results are shown in Table 7 below. Each of the removed adhesive preparations was used as a test sample for measuring availability in Test Example 8.

Table 7

| Example | AUC value for 7 days to removal (ng·h/mL) | AUC value for 14 days to day 7 after removal (ng·h/mL) |
|---|---|---|
| Example 1 | 1789.13 | 1887.68 |
| Example 6 | 21.84 | 22.47 |

As a result, the adhesive preparation Example 1 exhibited persistent transdermal absorption with Tmax on day 4 and showed a high AUC value. It was revealed that the application period is 7 days at a maximum, preferably 4 days.

Test Example 4: Rabbit Skin Primary Irritation Test

Male Japanese albino (Kbl: JW) rabbits (9 weeks of age, SPF) were used for this test. Rabbits having a smooth skin without abnormalities in body weight change and in general conditions in a quarantine and accommodation period were selected and subjected to the test. On the day (24 hours) before application, the hair on the back was shaved using an electric hair clipper, to prepare 4 application sites in total at both sides of the midline. Among them, 2 sites served as normal skin, while the other 2 sites served as wounded skin prepared by scarring the stratum corneum with a 23G injection needle. The number of animals used was 3 in each group. Each of the adhesive preparations prepared in Example 1 and Comparative Example 4 was applied to the normal skin and the wounded skin, and then the application sites were blocked up for 12 hours, 24 hours or 48 hours. Regarding the application procedure, each of the adhesive preparations prepared in Example 1 and Comparative Example 4 was stuck to the application sites and fixed with a nonwoven fabric adhesive bandage (Meshpore, No. 50 (trade name), manufactured by Nichiban Co., Ltd.). Then, the whole of the application sites was covered with a gauze, wrapped with an elastic adhesive bandage (Elastpore, No. 100 (trade name), manufactured by Nichiban Co., Ltd.), and blocked up for each period. The adhesive preparation in Comparative Example 4 was used as a negative control preparation which was the same composition as the preparation in Example 1 except for minodronic acid hydrate and sodium hydroxide. After blockage for each period, the adhesive elastic bandage etc. was removed, and the remaining test preparation and control preparation were gently wiped off with absorbent cotton moistened with warm water. 30 minutes, 24 hours and 48 hours after removal, the skin reaction was checked, and the average score for each preparation was determined according to the evaluation criterion table 3

according to Draize test (see J. Pharmacol. Exp. Ther., 83, 377-390 (1944)) shown in Table 8.

From the average scores per animal at removal and 48 hours after removal, the average score for each preparation was calculated to determine primary irritation index (P.I.I.). P.I.I. = total of average scores per animal for each preparation /3 (rabbits)

Safety levels were set as follows: (i) no irritancy when P.I.I., is 0, (ii) slight irritancy when it is more than 0 but 2 or less, (iii) moderate irritancy when it is more than 2 but 5 or less, and (iv) severe irritancy when it exceeds 5. When P.I.I. is 0 or more than 0 but 2 or less, it can be assessed that there is no problem on the irritancy in accordance with the safety levels.

Table 8

| A. Erythema and eschar formation | Score |
|---|---|
| No erythema | 0 |
| Very slight erythema (barely perceptible) | 1 |
| Definite erythema | 2 |
| Moderate to severe erythema | 3 |
| Severe erythema with deep redness and slight eschar formation(lesion that reaches deep into the skin) | 4 |
| B. Edema formation | |
| No edema | 0 |
| Very slight edema (barely perceptible) | 1 |
| Definite edema (distinct from the surrounding) | 2 |
| Moderate to severe edema (raised approx. 1 mm) | 3 |
| Severe edema (raised more than 1 mm and extending beyond the surrounding) | 4 |

The results are shown in Table 9 below.

Table 9

| Application period | Plaster | P.I.I. |
|---|---|---|
| 12 hours | Comparative example 4 | 0.08 |
| | Example 1 | 0.58 |
| 24 hours | Comparative example 4 | 0.58 |
| | Example 1 | 0.92 |
| 48 hours | Comparative example 4 | 0.75 |
| | Example 1 | 0.83 |

As a result, the adhesive preparations of the present invention showed P.I.I. of 1 or less during each application period, and thus were found to have very slight irritancy.

Test Example 5: Adhesion Test (measurement of 180° peel adhesion)

180° peel adhesion against a test plate was measured in the following method in accordance with JIS Z 0237 (test method for adhesive tapes and sheets).

In the test method, a phenol resin plate (Product No. FL-FLE, manufactured by Futamura Chemical Co., Ltd.), as a test plate, and a 10 mm-wide test specimen were used. A tensile testing machine (RT-3020D-CW) manufactured by RheoTec was used.

Each test specimen cut out 10 mm wide was stuck to the phenol resin plate so as not to trap air between the two, and was pressure-bonded to the test plate by rolling a roller weighing 850 g back and forth twice thereon at a rate of 20 mm/s. This was let to stand at 37°C for 30 minutes. One edge of the test specimen was folded back at 180° and fixed to the upper part of the testing machine. Meanwhile, the lower part of the test plate was fixed to the testing machine. Then, the test specimen was peeled off continuously at a rate of 200 mm/min, to determine the load with which it was peeled off.

The results are shown in Table 10 below. As a result, any of the adhesive preparations of the present invention

showed suitable adhesion.

Table 10

| | Results of tensile test (g weight) |
|---|---|
| Example 1 | 1.1 |
| Example 2 | 63.4 |
| Example 3(1) | 69.2 |
| Example 3(2) | 93.1 |
| Example 3(3) | 21.1 |
| Example 4 | 16.8 |
| Example 5 | 21.1 |

Test Example 6: Adhesion Test (probe tack test)

The probe tack test was conducted in the following method in accordance with the method ASTM D2979.
Each test specimen was stuck with the adhesive surface facing up to a specimen-fixing plastic plate with a hole in the center. Then, the plastic plate with the test specimen was set to a tensile testing machine (RT-3020D-CW manufactured by RheoTec). The specimen-fixing plate with the test specimen stuck thereto was moved upward at a rate of 200 mm/min and pressure-bonded to an acrylic resin cylinder with a diameter of 10 mm under a load of 200 g. Immediately the test specimen was pulled off in the vertical direction at a rate of 200 mm/min, to determine the load with which the specimen was pulled off.
The results are shown in Table 11 below.
As a result, any of the adhesive preparations of the present invention showed suitable adhesion.

Table 11

| | Results of probe tack test (g weight) |
|---|---|
| Example 1 | 302.7 |
| Example 2 | 420.0 |
| Example 3(1) | 541.2 |
| Example 3(2) | 541.2 |
| Example 3(3) | 705.7 |
| Example 4 | 297.2 |
| Example 5 | 284.4 |

Test Example 7: Release Test

Each test adhesive preparation was mounted in a two-chamber diffusion cell, and a phosphate buffer, pH 7.4, was poured into the receiver side of the cell. The temperature was kept constant by circulating water at 32°C around the cell. The phosphate buffer (1000 μL) was periodically collected from the cell, and immediately the same volume of buffer was added to the cell for replenishment. The concentration of minodronic acid was measured over a period of 8 hours by HPLC under the following conditions, and the cumulative permeated amount of the active ingredient was determined.

<HPLC Conditions>

Column: Develosil ODS-5-HG (manufactured by Nomura Chemical Co., Ltd.)
Column temperature: 35°C
Mobile phase: 4.46 g of pyrophosphoric acid·decahydrate was dissolved in about 950 mL of water. To this was

added 2.5 mL of a 25% solution of hexadecyl trimethyl ammonium hydroxide in methanol, followed by water to adjust the total volume to 1000 mL. The pH was adjusted to 7.5 with phosphoric acid. Then, 150 mL of acetonitrilewas added to 850 mL of the resulting solution to prepare the mobile phase.
Detection wavelength: excitation wavelength 230 nm, absorption wavelength 397 nm.
Injection volume: 5 μL
Retention time: about 10 min.

The results are shown in Table 12. As a result, the adhesive preparations of the present invention released the active ingredient at a rate ranging from about 40%/8 hr to about 100%/8 hr, which is considered good in releasability. In particular, these adhesive preparations showed 50%/8 hr or more, and thus were found to be extremely excellent in releasability of active ingredient.

Table 12

| | Cumulative released amount for 8 hours (%) |
|---|---|
| Example 1 | 100.17 |
| Example 4 | 56.28 |
| Example 5 | 54.09 |

Test Example 8: Availability Measurement Test

Each of the adhesive preparations which were applied to male hairless rats and then removed in the in vivo transdermal absorption test in Test Example 3 was measured for the remaining amount of the active ingredient therein by the following method. From the remaining amount of the active ingredient, the residual ratio was calculated. The results are shown in Table 13.

<Method of recovery from the adhesive preparations>

15 mL of tetrahydrofuran (THF) per adhesive preparation of the present invention was poured into the container. After 30 minutes of shaking, 25 mL of an eluent (mobile phase) was poured thereto. After further 20 minutes of shaking, the solution in the container was recovered. Then, 15-minute shaking of the adhesive preparation in additional 25 mL of an eluent, followed by recovering the eluent was conducted twice. The total volume of the recovered solution was adjusted accurately to 100 mL with an eluent. The resulting solution was filtered through a membrane filter with a pore diameter of 0.45 μm (Millex-HV manufactured by MILLIPORE). About 5 ml of the first filtrate was discarded. 4 mL of the next filtrate was accuratelymeasured, and 5 mL of an internal standard solution was accurately added thereto. The total volume was adjusted to 25 mL with an eluent.
In the thus obtained solution, the amount of minodronic acid was measured by HPLC under the following conditions, and its amount in terms of hydrate was determined as the remaining amount of the active ingredient.

<HPLC Conditions>

Column: Develosil ODS-5-HG (manufactured by Nomura Chemical Co., Ltd.)
Column temperature: 35°C
Eluent: 4.46 g of pyrophosphoric acid·decahydrate was dissolved in about 950 mL of water. To this was added 2.5 mL of a 25% solution of hexadecyl trimethyl ammonium hydroxide in methanol, followed by water to adjust the total volume to 1000 mL. The pH was adjusted to 7.5 with phosphoric acid. Then, 150 mL of acetonitrile and 1 mL of THF were added to 850 mL of the resulting solution to prepare the eluent.
Detection wavelength: excitation wavelength 230 nm, absorption wavelength 397 nm.
Injection volume: 5 μL
Retention time: about 10 min.

As a result, as shown in Table 13 below, both of adhesive preparations of the present invention showed availability as high as 20% or more (i.e., low residual ratio).

Table 13

| | Residual ratio of active ingredient (%) | Availability (%) |
|---|---|---|
| Example 1 | 25.5 | 74.5 |
| Example 6 | 77.3 | 22.7 |
| Availability = 100 - Residual ratio | | |

Test Example 9: Stability Test

The adhesive preparation in Example 1 was stored for 6 months at room temperature and examined for change in the content of the active ingredient. The content of the active ingredient was measured in the same operation as described in Test Example 8. The results are shown in Table 14 below.

As a result, the adhesive preparation of the present invention showed little change in the content of the active ingredient after the predetermined period of storage and was found to be a stable adhesive preparation.

Table 14

| | Residual ratio of active ingredient (%) |
|---|---|
| Example 1 | 99.7 |

Test Example 10: Coatability Test

Ten preparations each (10 $cm^2$ each) selected at random from the adhesive preparations obtained in Example 1, 2, 4 or 5 were measured for their mass. The theoretical mass of the support and the release paper was subtracted from the mass of each adhesive preparation, to determine the mass of the plaster layer. The difference (deviation %) between the mean mass of the plaster layers and the mass of the individual plaster layer was determined to calculate the relative standard deviation. The results are shown in Table 15 below.

Table 15

| | Relative standard deviation (%) |
|---|---|
| Example 1 | 2.6 |
| Example 2 | 4.1 |
| Example 4 | 4.3 |
| Example 5 | 3.2 |
| Example 7(1) | 2.4 |
| Example 7(2) | 2.8 |
| Example 7(3) | 2.3 |
| Example 7(4) | 3.8 |
| Example 8 | 4.7 |
| Example 9(1) | 3.4 |
| Example 9(2) | 4.7 |
| Example 9(3) | 3.9 |
| Example 9(4) | 2.7 |
| Example 10 | 4.4 |
| Example 11(1) | 3.2 |
| Example 11(2) | 2.8 |
| Example 11(3) | 4.8 |

(continued)

| | Relative standard deviation (%) |
|---|---|
| Example 11(4) | 1.4 |
| Comparative example 2 | unmeasurable |

As a result, any of the adhesive preparations of the present invention showed relative standard deviation of 5% or less, and thus showed good coatability.

Industrial Applicability

According to the present invention, there is provided an adhesive preparation which can sustain the transdermal absorption of the bisphosphonic acid derivative or the like (for example, minodronic acid, zoledronic acid, ibandronic acid, salts of any of the foregoing compounds or hydrates of any of the foregoing compounds) into the body in an efficient manner with causing less skin irritation. The adhesive preparation of the present invention having a high AUC value can sustain such a sufficient blood drug concentration as to exhibit drug effect even by intermittent administration. The adhesive preparation of the present invention also can provide improved patients' compliance with the medication and can exhibit drug effect over a further prolonged period, as compared with oral administration. Thus, the adhesive preparation of the present invention is extremely useful. The adhesive preparation of the invention can also be industrially produced because the plaster layer therein is excellent in coatability.

## Claims

1. An adhesive preparation having a plaster layer disposed on a support, which is **characterized by** that the adhesive preparation comprises at least one active ingredient selected from the group consisting of a bisphosphonic acid derivative, a salt thereof and a hydrate of either of the bisphosphonic acid derivative or the salt, a solubilizer for the active ingredient, propyleneglycol, ahydrogenatedterpeneresin, an adhesive base, and a softening agent in the plaster layer.

2. The adhesive preparation according to Claim 1, wherein the softening agent is at least one member selected from the group consisting of polybutene, liquid paraffin, and light liquid paraffin.

3. The adhesive preparation according to Claim 1, wherein the content of the active ingredient in one adhesive preparation or in the adhesive preparations applied once is 0.01 mg to 30 mg.

4. The adhesive preparation according to Claim 3, wherein the sum of an area under blood concentration-time curve (AUC) value of the bisphosphonic acid derivative for 1 month is 5 ng·hr/mL to 5 μg·hr/mL.

5. The adhesive preparation according to Claim 1, wherein the relative standard deviation of the mass of the plaster layer in one adhesive preparation is 5% or less when the number of samples is 10.

6. The adhesive preparation according to Claim 1, wherein the thickness of the plaster layer is 20 μm to 300 μm.

7. The adhesive preparation according to Claim 1, wherein the active ingredient is at least one member selected from the group consisting of minodronic acid, alendronic acid, ibandronic acid, zoledronic acid, risedronic acid, incadronic acid, etidronic acid, olpadronic acid, clodronic acid, tiludronic acid, neridronic acid, pamidronic acid, salts of any of the foregoing compounds and hydrates of any of the foregoing compounds.

8. The adhesive preparation according to Claim 1, wherein the active ingredient is at least one member selected from the group consisting of alendronic acid, ibandronic acid, zoledronic acid, risedronic acid, salts of any of the foregoing compounds and hydrates of any of the foregoing compounds.

9. The adhesive preparation according to Claim 1, wherein the active ingredient is minodronic acid, a salt thereof or a hydrate of either of minodronic acid or the salt.

10. The adhesive preparation according to Claim 1, which further comprises at least one member selected from the

group consisting of polyoxyethylene alkyl ether, polyoxyethylene alkyl ether phosphate or a salt thereof, oleyl alcohol, and sorbitan fatty acid ester.

**11.** The adhesive preparation according to Claim 10, wherein the polyoxyethylene alkyl ether is polyoxyethylene behenyl ether, the polyoxyethylene alkyl ether phosphate is tripolyoxyethylene cetyl ether phosphate, and the sorbitan fatty acid ester is sorbitan sesquioleate.

**12.** The adhesive preparation according to Claim 2, which further comprises isopropyl myristate.

**13.** The adhesive preparation according to Claim 1, which further comprises at least one member selected from the group consisting of a rosin resin, a petroleum resin, and another terpene resin.

**14.** The adhesive preparation according to Claim 13, wherein the rosin resin is glycerol ester of hydrogenated rosin and/or super light-colored rosin ester.

**15.** The adhesive preparation according to Claim 1, wherein the adhesive base is at least one member selected from the group consisting of a rubber adhesive base, an acrylic adhesive base, and a silicone adhesive base.

**16.** The adhesive preparation according to Claim 15, wherein the rubber adhesive base is at least one member selected from the group consisting of a styrene-isoprene-styrene block copolymer, a styrene-butadiene-styrene block copolymer, a styrene-ethylene-styrene block copolymer, a styrene-butylene-styrene block copolymer, polyisoprene, polyisobutylene, and an ethylene-vinyl acetate copolymer.

**17.** The adhesive preparation according to Claim 15, wherein the rubber adhesive base is a styrene-isoprene-styrene block copolymer.

**18.** The adhesive preparation according to Claim 1, wherein 0.1 to 10 parts by mass of the active ingredient, 1 to 50 parts by mass of propylene glycol, 10 to 80 parts by mass of the adhesive base, 5 to 60 parts by mass of the hydrogenated terpene resin, and 5 to 60 parts by mass of the softening agent are contained in 100 parts by mass of the plaster layer.

**19.** The adhesive preparation according to Claim 18, wherein the adhesive base is a rubber adhesive base and is contained in an amount of 10 to 50 parts by mass, and the hydrogenated terpene resin is contained in an amount of 10 to 60 parts by mass.

**20.** The adhesive preparation according to Claim 18, wherein the adhesive base is an acrylic adhesive base and is contained in an amount of 20 to 80 parts by mass, and the hydrogenated terpene resin is contained in an amount of 5 to 30 parts by mass.

**21.** The adhesive preparation according to Claim 18, wherein the adhesive base is a silicone adhesive base and is contained in an amount of 20 to 80 parts by mass, and the hydrogenated terpene resin is contained in an amount of 5 to 30 parts by mass.

**22.** The adhesive preparation according to Claim 1, wherein the solubilizer for the active ingredient is water or an aqueous alkaline solution.

**23.** The adhesive preparation according to Claim 1, which is applied once a day, once every two days, once every 3 to 4 days, once a week to once every three weeks, or once a month to once every three months and is stuck for 6 hours to 7 days after being once applied.

**24.** The adhesive preparation according to Claim 1, which is applied once a day, once every two days, once a week, or once a month and is stuck for 24 hours to 4 days after being once applied.

**25.** The adhesive preparation according to Claim 1, which is a preventive and/or therapeutic preparation for at least one diseaseselectedfrom osteoporosis,bone metastasisfrom cancer, hypercalcemia, multiple myeloma, Paget's disease, periodontal diseases, osteoarthritis, and rheumatoid arthritis.

**26.** An adhesive preparation containing 0.5 to 5 parts by mass of minodronic acid, a salt thereof or a hydrate of either

of minodronic acid or the salt as an active ingredient, 2 to 15 parts by mass of a combination of propylene glycol and polyoxyethylene behenyl ether, 20 to 60 parts by mass of a combination of a hydrogenated terpene resin and glycerol ester of hydrogenated rosin, 10 to 40 parts by mass of polybutene, liquid paraffin or light liquid paraffin, and 15 to 35 parts by mass of a styrene-isoprene-styrene block copolymer in 100 parts by mass of a plaster layer,
the adhesive preparation satisfying any of the following conditions (1) to (3):

(1) the content of the active ingredient in one adhesive preparation or in the adhesive preparations applied once is 0.1 mg to 10 mg,
(2) the sum of an area under blood concentration-time curve (AUC) value of minodronic acid for 1 month is 30 ng·hr/mL to 5 μg·hr/mL, and
(3) the relative standard deviation of the mass of the plaster layer in one adhesive preparation is 5% or less when the number of samples is 10.

**27.** An adhesive preparation containing 0.5 to 5 parts by mass of minodronic acid, a salt thereof or a hydrate of either of minodronic acid or the salt as an active ingredient, 2 to 15 parts by mass of a combination of propylene glycol and polyoxyethylene behenyl ether, 20 to 60 parts by mass of a combination of a hydrogenated terpene resin and glycerol ester of hydrogenated rosin, 10 to 40 parts by mass of polybutene, liquid paraffin or light liquid paraffin, and 15 to 35 parts by mass of a styrene-isoprene-styrene block copolymer in 100 parts by mass of a plaster layer,
the adhesive preparation satisfying any of the following conditions (1) to (3):

(1) the content of the active ingredient in one adhesive preparation or in the adhesive preparations applied once is 0.1 mg to 10 mg,
(2) the sum of an area under blood concentration-time curve (AUC) value of minodronic acid for 1 month is 30 ng·hr/mL to 500 ng·hr/mL, and
(3) the relative standard deviation of the mass of the plaster layer in one adhesive preparation is 5% or less when the number of samples is 10.

**28.** Use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent as an adhesive preparation.

**29.** Use of a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent for the production of an adhesive preparation.

**30.** A method of applying, to the skin, a composition comprising at least one active ingredient selected from the group consisting of a bisphosphonic acid derivative, a salt thereof and a hydrate of either of the bisphosphonic acid derivative or the salt, a solubilizer for the active ingredient, propylene glycol, a hydrogenated terpene resin, an adhesive base, and a softening agent.

Fig. 1

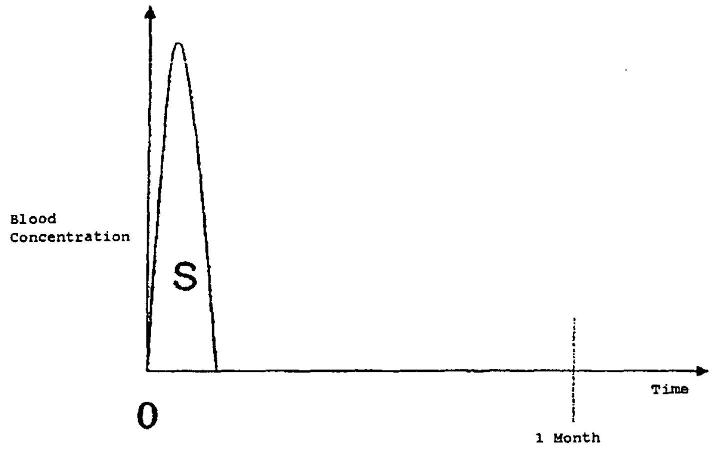


Fig. 2

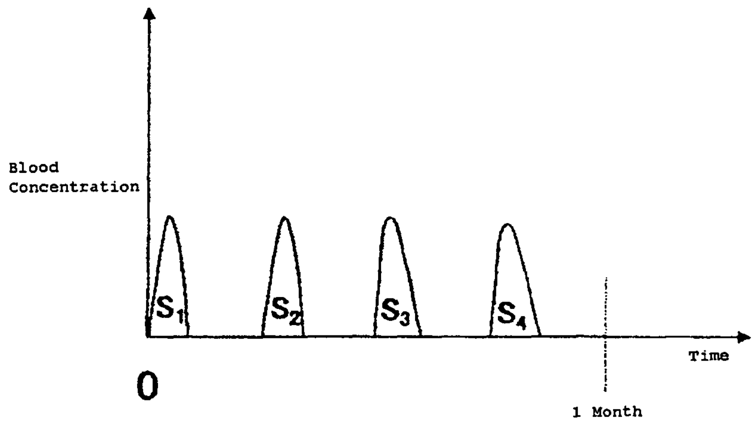


Fig. 3

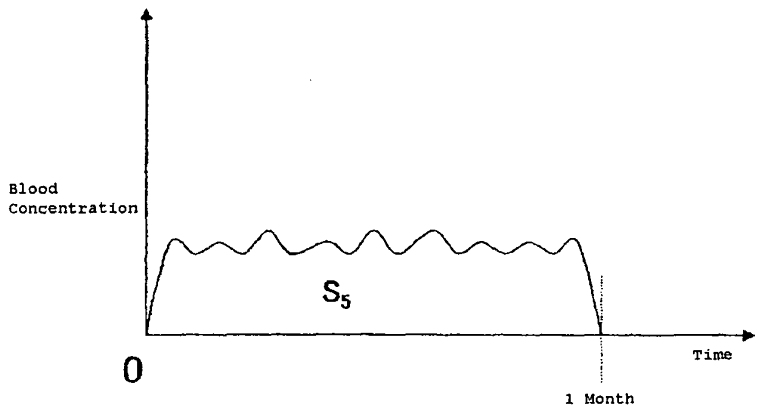

## INTERNATIONAL SEARCH REPORT

International application No.
PCT/JP2007/067597

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
A61K31/663, A61K9/70, A61K47/06, A61K47/08, A61K47/10, A61K47/14, A61K47/32, A61P1/02, A61P3/14, A61P19/00, A61P19/02, A61P19/10, A61P29/00, A61P35/00, A61P35/02, A61P35/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CA(STN), REGISTRY(STN), BIOSIS(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| Y | JP 2004-250330 A (ONO PHARM CO., LTD.), 09 September, 2004 (09.09.04), Examples 3 to 9 & WO 2003/068241 A1 & JP 2004-250423 A & EP 1475095 A1 & US 2005/106186 A1 | 1-27,29 |
| Y | JP 2003-063955 A (NICHIBAN KABUSHIKI KAISHA), 05 March, 2003 (05.03.03), Table 1 (Family: none) | 1-27,29 |
| A | Edited by Japan Pharmaceutical Association, Revised edition No.12, Chozai Shishin, 08 September, 2006 (08.09.06) 3rd edition, Kabushiki Kaisha Yakuji Nipposha Hakko, page 150 | 1-27,29 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 November, 2007 (16.11.07) | 27 November, 2007 (27.11.07) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2007/067597

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-137773 A (HISAMITSU PHARM CO., LTD.), 14 May, 2003 (14.05.03), & WO 2003/037393 A1 & EP 1462121 A1 & US 2005/042269 A1 | 1-27,29 |
| A | JP 2006-213658 A (HISAMITSU PHARM CO., LTD.), 17 August, 2006 (17.08.06), (Family: none) | 1-27,29 |

Form PCT/ISA/210 (continuation of second sheet) (April 2007)

**INTERNATIONAL SEARCH REPORT**

International application No.
PCT/JP2007/067597

Continuation of A. CLASSIFICATION OF SUBJECT MATTER
(International Patent Classification (IPC))

*A61K31/663*(2006.01)i, *A61K9/70*(2006.01)i, *A61K47/06*(2006.01)i, *A61K47/08*(2006.01)i, *A61K47/10*(2006.01)i, *A61K47/14*(2006.01)i, *A61K47/32*(2006.01)i, *A61P1/02*(2006.01)i, *A61P3/14*(2006.01)i, *A61P19/00*(2006.01)i, *A61P19/02*(2006.01)i, *A61P19/10*(2006.01)i, *A61P29/00*(2006.01)i, *A61P35/00*(2006.01)i, *A61P35/02*(2006.01)i, *A61P35/04*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2007/067597

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 28, 30
because they relate to subject matter not required to be searched by this Authority, namely:
Claims 28 and 30 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the
☐ The additional search fees were accompanied by the applicant's protest and, where applicable, payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 6099457 B
- US 5133972 A
- JP 53034930 A
- WO 03068241 A

### Non-patent literature cited in the description

- *J. Pharmacol. Exp. Ther.,* 1944, vol. 83, 377-390